# EUROPEAN PATENT APPLICATION

(11) **EP 2 098 176 A2**
(43) Date of publication of application: **09.09.2009**
(21) Application number: 09003126.1
(22) Date of filing: 04.03.2009
(51) Int. Cl.: A61B 17/128, A61B 17/122

(54) **Magazine type clipping device**

(30) Priority: 06.03.2008 JP 2008055990; 06.03.2008 JP 2008056395; 29.09.2008 JP 2008250483
(71) Applicant: FUJIFILM Corporation, Minato-ku Tokyo (JP)
(72) Inventor: Cui, Shengfu, Kanagawa (JP); Iida, Takayuki, Kanagawa (JP)
(74) Representative: Höhfeld, Jochen

(57) **Abstract**

A magazine type clipping device (10) comprises: clips (12) loaded into a sheath (16) while being engaged with other clips longitudinally connected together; at least one connection ring (14) fitted into the sheath (16) so as to be capable of advancing and retreating, and covering an engagement portion of the clips (12) to maintain the clips (12) in a connected state; and a manipulating wire (20) connected to a rearmost clip and adapted to pull a clip row formed of the plurality of clips (12), each clip (12) having a pair of openable/closable claw portions (22) and a tail portion, the connection ring (14) having a connection maintaining region retaining a pair of clips engaged with each other, with the pair of claw portions (22) of a succeeding clip being closed while holding the tail portion of a preceding clip.

## Description

### BACKGROUND OF THE INVENTION

The present invention relates to an endoscopic clipping device used for stopping bleeding, closing a puncture, etc. in a living body or the like and, in particular, to a magazine type clipping device which allows a plurality of clips to be used in succession.

An endoscopic clipping device causes a clip to protrude from the forward end of an endocope inserted into a living body to pinch a bleeding portion or a portion to be treated after the removal of the lesion tissue with the clip, thereby stopping the bleeding or closing the puncture. In a conventionally used endoscopic clipping device, a single clip is detachably attached to the forward end of a manipulating wire, and each time clipping is performed, the entire sheath is drawn out of the endoscope, and the sheath is loaded with the next clip before being inserted into the endoscope again for the next clipping. In this way, the convention clipping device involves a rather bothersome operation.

In this connection, there has been proposed an endoscopic clipping device allowing successive clipping. For example, JP 2006-187391 A discloses an endoscopic clipping device in which a connection hole formed in the rear end portion of a preceding clip is engaged with forward end claw portions of a succeeding clip, whereby a plurality of clips are directly connected together, with their orientations being alternately changed by 90 degrees. Further, JP 2006-087537 A discloses an endoscopic clipping device in which outwardly bent connection claws are formed at the rear end portions of a pair of plate spring members forming a clip, with engagement holes to be engaged with the connection claws of a preceding clip being formed in the vicinity of the forward ends of the plate spring members. In the endoscopic clipping device, in a state in which a succeeding clip has been made narrower, the connection claws at the rear end of the preceding clip are inserted into the engagement holes in the vicinity of the forward end of the succeeding clip for locking, whereby a plurality of clips oriented in the same direction are connected directly to each other.

By using the devices as disclosed in JP 2006-187391 A and JP 2006-087537 A, it is possible to perform successive clipping operations. In those conventional devices, however, the clips are maintained in the connected state solely through engagement between the preceding and succeeding clips, with the connecting portion being exposed in the sheath. Thus, the connection is rather unstable, and disconnection may occur at the time of insertion into the endoscope or when the devices is passed through a curved portion, or there is the possibility of an excessive force being applied to the connecting portion to cause twisting or distortion in the clips to be used. When the clips are disconnected within the sheath, the clips are dropped about from the forward end of the sheath. Further, there is a fear of projections or corner portions of the clips at the connecting portion damaging the inner wall of the sheath when a curved portion is passed through.

Further, in the devices as disclosed in JP 2006-187391 A and JP 2006-087537 A, the clips are accommodated in the sheath to thereby maintain the clips in the closed state, and at the time of clipping operation, the clips are caused to advance and retreat within the sheath while pressing the sheath inner wall with the resilient force of the clips. Thus, the clip pulling load is unnecessarily large, and there is a fear of the sheath inner wall being damaged. Further, in those conventional devices, the connection is maintained solely through engagement between the front and rear clips, and the advancing/retreating movement due to the manipulating wire is transmitted by the connected portion. However, this connected portion involves play, and hence wobble is generated at the time of advancing/retreating movement, thus giving the operator an unstable feel, and further, a precise control of the advancing/retreating movement is rather difficult to perform.

### SUMMARY OF THE INVENTION

It is an object of the present invention to eliminate the above-mentioned problems in the related art and to provide a magazine type clipping device which is capable of reliably maintaining the connected state of the clips loaded in such a state, which is free from a fear of damaging the sheath inner wall, and which can perform clipping operation smoothly and with high precision. Another object of the present invention is to provide a magazine type clipping device capable of effecting the clamping and separation of (canceling of connection) the foremost clip by a series of operations in one direction.

A magazine type clipping device according to a first aspect of the present invention comprises: a plurality of clips loaded into a forward end portion of a sheath while being engaged with other clips longitudinally connected together; at least one connection ring fitted into the sheath so as to be capable of advancing and retreating, and covering an engagement portion of the clips to maintain the clips in a connected state; and a manipulating wire connected to a rearmost clip and adapted to pull a clip row formed of the plurality of clips, in which each of the plurality of clips has a pair of openable/closable claw portions and a tail portion, in which the connection ring has a connection maintaining region retaining a pair of the plurality of clips longitudinally engaged with each other, with the pair of claw portions of a succeeding clip being closed while holding the tail portion of a preceding clip, and in which the engagement portion between the pair of the plurality of clips is detached from the connection maintaining region through an operation of pulling the manipulating wire to open the pair of claw portions of the succeeding clip, with the tail portion of the preceding clip being detached from therebetween.

A magazine type clipping device according to a second aspect of the present invention comprises: a plurality of clips loaded into a forward end portion of a sheath while being engaged with other clips longitudinally connected together; at least one connection ring fitted into the sheath so as to be capable of advancing and retreating, and covering an engagement portion of the clips to maintain the clips in a connected state; and a manipulating wire connected to a rearmost clip and adapted to pull a clip row formed of the plurality of clips, in which the connection ring has at least one slit extending from a proximal end thereof to a position on a proximal end side of the engagement portion between the plurality of clips.

### BRIEF DESCRIPTION OF THE DRAWINGS

In the accompanying drawings:
FIGS. 1A and 1B are a side sectional view and a front sectional view, respectively, illustrating a clipping device according to Embodiment 1 of the present invention;
FIG. 2 is a perspective view of a clip used in Embodiment 1;
FIGS. 3A through 3C are a front view, a front sectional view, and a bottom view of a connection ring used in Embodiment 1;
FIGS. 4A and 4B are partial sectional views illustrating how clips are maintained in a connected state by a connection ring;
FIG. 5 is an enlarged sectional view illustrating the relationship between the clip, the connection ring, and the sheath of Embodiment 1;
FIGS. 6A through 6E are sectional views illustrating stepwise the state of the clipping device according to Embodiment 1 during clipping manipulation;
FIGS. 7A through 7C are a front view, a front sectional view, and a bottom view of a connection ring used in Embodiment 2;
FIGS. 8A through 8C are partial sectional views illustrating stepwise the state of the clips and the connection ring during clipping manipulation of Embodiment 2;
FIG. 9 is an enlarged sectional view of a lower end portion of the connection ring of Embodiment 2;
FIGS. 10A through 10E are sectional views illustrating stepwise the state of the clipping device of Embodiment 2 during clipping manipulation;
FIGS. 11A through 11C are a front view, a front sectional view, and a bottom view of a connection ring used in Embodiment 3;
FIG. 12 is a schematic diagram illustrating the positional relationship between skirt portions of the connection ring and the clip used in Embodiment 3;
FIGS. 13A through 13C are a front view, a front sectional view, and a bottom view of a connection ring used in a modification of Embodiment 3;
FIG. 14 is a schematic diagram illustrating the positional relationship between skirt portions of the connection ring and the clip used in the modification of Embodiment 3;
FIGS. 15A and 15B are a perspective view and a partial enlarged view of a clip used in Embodiment 4;
FIGS. 16 and 17 are a perspective view and a sectional view of a manipulating portion used in Embodiment 5;
FIG. 18 is a perspective view of the manipulating portion of Embodiment 5 with a slider guide removed therefrom;
FIG. 19A is a perspective view of a guide portion of the slider guide;
FIG. 19B is a schematic developed view of the slider guide;
FIG. 20 is a perspective view of a rotating position regulating member; and
FIG. 21 is a schematic developed view of the slider guide for illustrating how clipping manipulation is performed in Embodiment 5.

### DETAILED DESCRIPTION OF THE PREFERRED EMBODIMENTS

In the following, preferred embodiments of a clipping device of the present invention are described with reference to the accompanying drawings.

### Embodiment 1

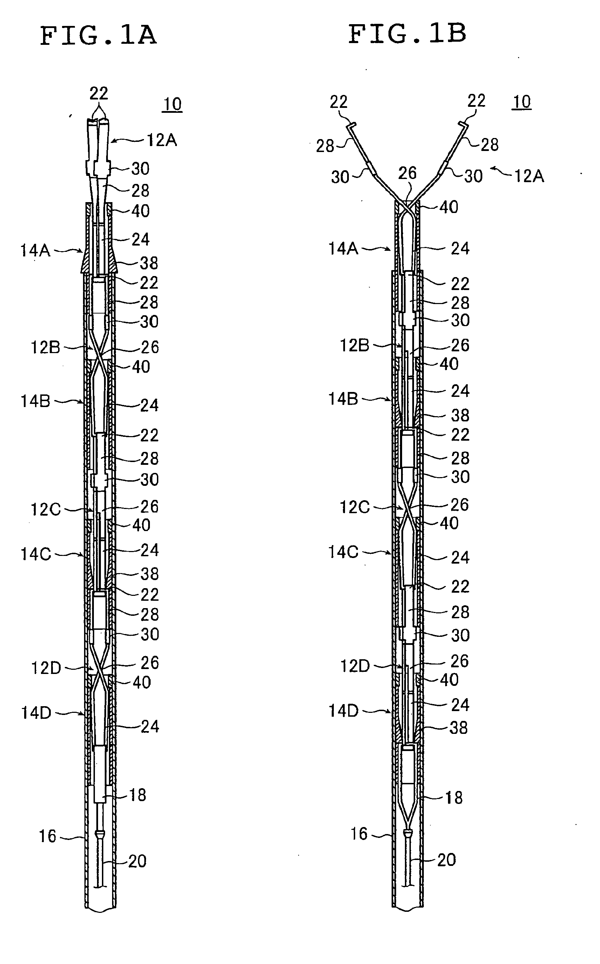

FIGS. 1A and 1B are sectional views of a clipping device 10 according to Embodiment 1 of the present invention, and FIG. 1B is a diagram as seen from an angle differing from FIG. 1A by 90 degrees.

The clipping device 10 is a magazine type clipping device in which clips can be used in succession and which has a plurality of clips 12 (12A, 12B, 12C, and 12D), a dummy clip 18 connected to the rearmost clip 12D, a manipulating wire 20 connected to the dummy clip, and connection rings 14 (14A, 14B, 14C, and 14D) covering the engagement portions of the adjacent clips 12 to maintain the clips 12 in the connected state, with those components being fitted into a sheath 16. FIGS. 1A and 1B illustrate an initial state immediately before the start of clipping manipulation by the foremost clip 12.

One clip 12 and one connection ring 14 corresponding to the clip 12 form one endoscopic bleeding stop clip member, and the clipping device 10 includes a plurality of such bleeding stop clip members loaded into the interior of the distal end portion of the elongated sheath 16. The terminal end of the successive bleeding stop clip members is engaged with the dummy clip 18, and the manipulating wire 20 extends to the proximal end of the sheath 16 to be connected to a manipulating portion described below. When the manipulating wire 20 is drawn out by a predetermined length from the manipulating portion, and the dummy clip 18 is moved in one direction by the predetermined length, the series of clips 12 move by the same amount, and the foremost clip 12 is clamped by the connection ring 14 retaining the same, whereby clipping for stopping bleeding, marking, etc. is effected by the foremost clip 12. When the clipping by the foremost clip 12 has been completed, the sheath 16 is pulled toward the manipulating portion side by a predetermined length, whereby the next clip 12 is placed in a usable state (standby state), thus making it possible to perform clipping successively.

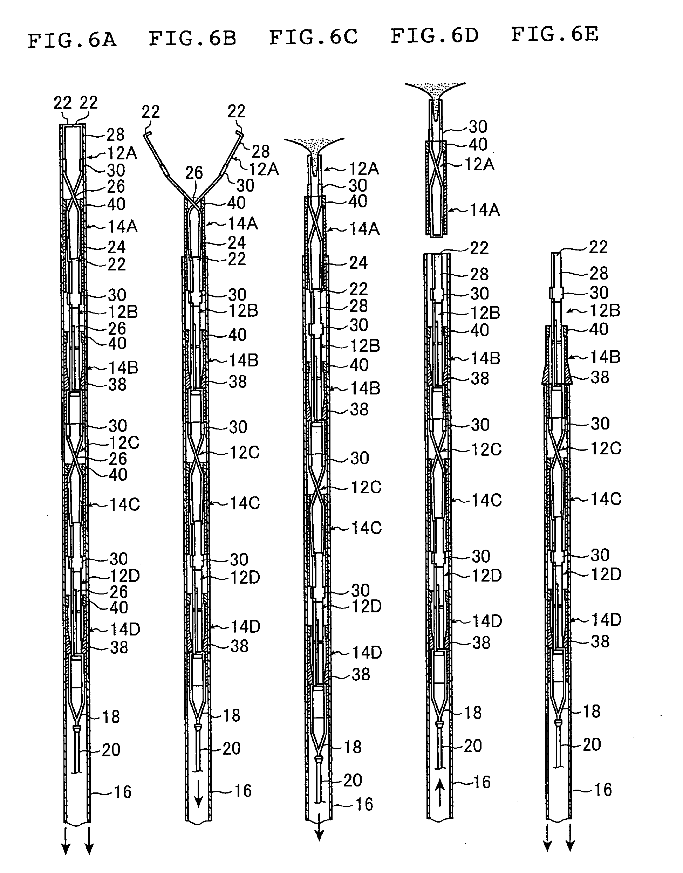

While in FIGS. 1A and 1B the foremost clip 12A protrudes from the distal end of the sheath 16, when loading the clips 12, etc. into the sheath 16, setting is effected such that the foremost clip 12A is completely accommodated within the sheath 16 as illustrated in FIG. 6A. Further, while in FIGS. 1A and 1B the number of clips 12 is four, that is, the clipping device is of a four-shooter type, it is possible for the clips 12 to be provided in any number not less than two.

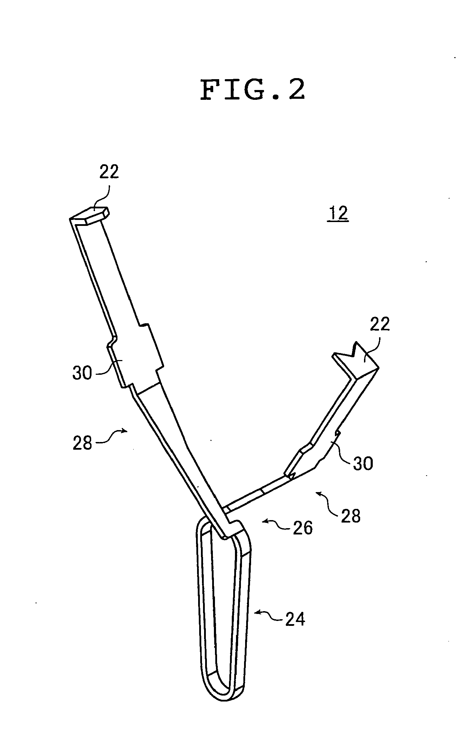

FIG. 2 is a perspective view of the clip 12. The clip 12 is a closed clip having a turned portion 24 turned by 180 degrees with respect to claw portions 22. That is, in forming the clip 12, a single elongated plate is bent by 180 degrees to form a closed end, and then both ends thereof are caused to cross each other. Further, the end portions are bent so as to be opposed to each other, thereby forming the claw portions 22 to two open ends. On the open-end side of the crossing portion 26, there exist armportions 28, and, on the closed-end portion thereof, there exists the turned portion 24. At the central portion of each arm portion 28, there is formed a partially widened projection 30. The clip 12 may be formed of a metal with biocompatibility. For example, it is possible to use SUS 631, which is a spring stainless steel.

In the clip 12, the forward end portion (a clamping portion 40 described below) of the connection ring 14 fitted onto the crossing portion 26 moves by a predetermined amount toward the claw portions 22 while pressurizing the arm portions 28, whereby the arm portions 28 and the claw portions 22 are closed, with the claw portions 22 exerting a predetermined fit-engagement force.

In order to reliably grasp an object, the claw portions 22 are formed as V-shaped male type and female type ones. Further, as illustrated in FIG. 2, the arm portions 28 of the clip 12 gradually increase in width from the crossing portion 26 toward the projections 30.

In the initial state illustrated in FIGS. 1A and 1B, the projections 30 are formed at positions where the length as measured from the forward end of the connection ring 14 is equal to or slightly larger than the requisite movement length for the clamping of the clip 12 by the connection ring 14. That is, the rear ends of the projections 30 are situated at the position of the forward end of the connection ring 14 or slightly on the forward end side thereof at the time of the completion of the clamping of the clip 12.

The projections 30 have a width larger than that of the portions of the distal end side opening and the proximal end side opening of the connection ring 14 abutted by the projections 30. Thus, while the portions of the clip 12 other than the projections 30 can enter the interior of the connection ring 14, the projections 30 cannot enter the interior either from the distal end side or the proximal end side of the connection ring 14.

As illustrated in FIGS. 1A and 1B, the claw portions 22 of the second clip 12B are engaged with the turned portion 24 of the first clip 12A and retained by the connection ring 14A in a closed state, whereby the first clip 12A and the second clip 12B are connected together. As illustrated in FIG. 1A, the claw portions 22 of the second clip 12B are engaged with the turned portion 24 of the first clip 12A in a direction orthogonal thereto, with the first clip 12A and the second clip 12B being differing in orientation by 90 degrees. Similarly, the clips 12C and 12D are connected together, with their orientations alternately differing by 90 degrees.

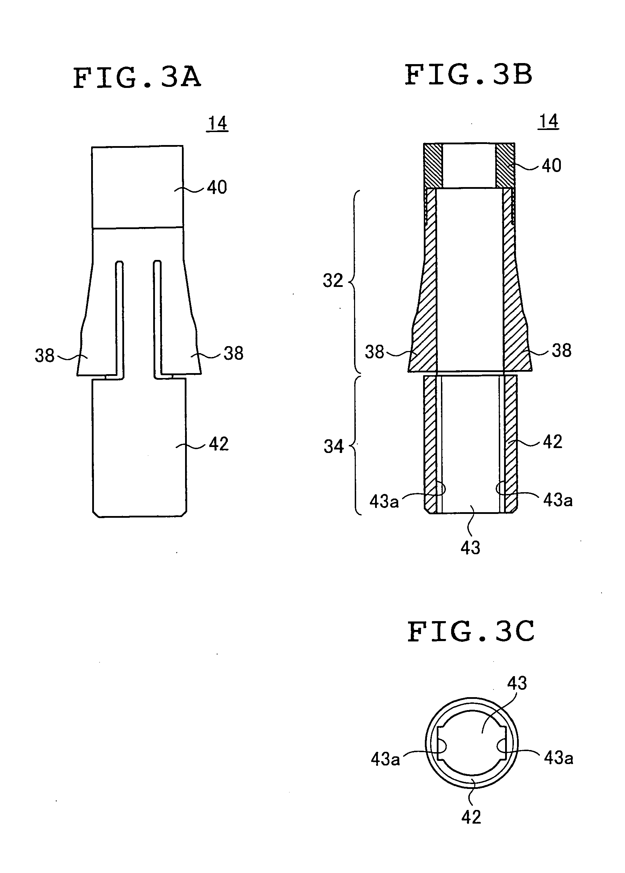

Each connection ring 14 is fitted into the sheath 16 so as to be capable of advancing and retreating while covering the engagement portion between two clips 12 and maintaining their connected state. That is, the outer diameter of the connection rings 14 is substantially the same as the inner diameter of the sheath 16 so that they can smoothly advance and retreat within the sheath 16 as the clips 12 move. FIGS. 3A through 3C schematically illustrate the construction of each connection ring 14. FIG. 3A is a front view of the connection ring 14, FIG. 3B is a sectional view thereof, and FIG. 3C is a bottom view thereof.

The connection ring 14 includes a clamping portion 40 and a retaining portion 42. In the connection ring 14, the clamping portion 40 formed of metal is fixed to the forward end of the retaining portion 42 formed of a resin, and the two members form an integral structure. The retaining portion 42 formed of a resin serves to maintain the connected state and to retain the clip within the connection ring, and the clamping portion 40 formed of metal serves to clamp the clip. It is also possible for the connection ring 14 to be formed by a single member if it can provide the functions of both the clamping portion 40 and the retaining portion 42.

The clamping portion 40 is a cylindrical (ring-like) metal component mounted to the forward end side of the connection ring 14, and has a hole whose inner diameter is larger than the width of the portion of the clip 12 in the vicinity of the crossing portion 26 and smaller than the width of the projections 30. Thus, while the clamping portion 40 can move in the vicinity of the crossing portion 26 of the clip 12 it retains, it cannot be detached to the forward end side beyond the projections 30. That is, the projections 30 function as a stopper determining the movement limit of the connection ring 14 advancing with respect to the clip 12.

The clamping portion 40 is set at a predetermined position in the vicinity of the crossing portion 26 of the clip 12. The clamping portion 40 moves from its initial position, i.e., from the crossing portion 26 toward the projections 30, with the arm portions 28 of the clip 12 increasing in width, whereby it closes the arm portions 28 of the diverging clip 12 to effect fixation and clamping. As the material of the clamping portion 40, there is used a metal with biocompatibility, for example, a stainless steel SUS 304. By forming the clamping portion 40 of metal, it is possible to exert on the metal clip 12 a frictional force, which serves as the clamping force.

The retaining portion 42 is a schematically cylindrical (ring-like) component formed by resin molding. The retaining portion 42 has a first region 32 retaining the preceding clip 12 and a second region 34 which is a connection retaining region retaining the next clip 12 while connected to the preceding clip.

The first region 32 has a large circular hole capable of accommodating the turned portion 24 of the clip 12 and larger than the hole of the clamping portion 40. On the outer surface of the forward end portion of the first region 32, there is formed a stepped portion onto which the clamping portion 40 is to be fitted, and the clamping portion 40 and the retaining portion 42 are fit-engaged with each other through close fit such that they are not detached from each other while loaded in the sheath 16 and during clipping manipulation. Further, the first region 32 has skirt portions 38 each diverging while inclined in a skirt-like fashion with respect to the axis of the connection ring 14 main body.

The forward end side, that is, the upper, base portion of the skirt portion 38 as seen in FIGS. 3A and 3B is connected to the main body of the retaining portion 42, whereas the lower, diverging portion thereof is partial ly separated f rom the main body to be radially diverged or closed. Two skirt portions 38 are formed so as to be separated from each other by 180 degrees at the same position in the pulling direction for the clip 12, that is, in the vertical direction in FIGS. 3A and 3B.

When left as they are, that is, when in a state in which no external force is being imparted thereto, the skirt portions 38 are diverged in a skirt-like fashion as illustrated in FIG. 3A. At this time, the interior of the first region 32 of the retaining portion 42 forms a columnar space as illustrated in FIG. 3B. When loading the connection rings 14 into the sheath 16, the following takes place: in the case, for example, of the second connection ring 14B illustrated in FIG. 1B, the skirt portions 38 are pushed in to enter the internal space, and the inner peripheral side portions of the skirt portions 38 pressurize the side surface (edge portion) of the turned portion 24 of the clip 12B retained by the first region 32, thus retaining the clip 12B such that it does not move in the rotating direction and the advancing/retreating direction within the connection ring 14B. It is also possible for the skirt portions 38 to pressurize and retain the clip retained by the second region 34, that is, the succeeding clip.

As in the case of the first connection ring 14A illustrated in FIG. 1A, the skirt portions 38 extend beyond the forward end of the sheath 16, and are opened due to their own elasticity, releasing the retention of the clip 12A and becoming wider than the inner diameter of the sheath 16 to prevent the connection ring 14A from retracting into the sheath 16. In this state, the manipulating wire 20 is pulled, and the clip 12A retreats, whereby the connection ring 14A advances relative to the clip 12A to clamp the clip 12A.

Thus, it is necessary for the skirt portions 38 to have elasticity so that they can be closed inwardly within the sheath 16 and widen in a skirt-like fashion when they get out of the forward end of the sheath 16 and are released from the external force. At the same time, it is also necessary for the skirt portions 38 to exhibit rigidity enabling them to retain the clip 12 within the sheath 16 and to withstand the repulsive force of the clamping force of the clip 12 at the forward end of the sheath 16.

From the above-mentioned viewpoints, as the material of the retaining portion 42, there is used a material exhibiting biocompatibility and providing the requisite elasticity and rigidity for the skirt portions 38. As for their configuration, it is determined so as to satisfy the requirements in terms of elasticity and rigidity for the skirt portions 38. As the material of the retaining portion 42, it is possible to use, for example, polyphenylsulfone (PPSU). From the viewpoint of ease of production, it is desirable for the retaining portion 42 to be formed as an integral molding.

The second region 34 is provided on the proximal end side of the first region 32. The succeeding clip 12 engaged with the clip 12 retained by the first region 32 is retained in a state in which the claw portions 22 thereof are closed while holding the closed end (tail portion) of the turned portion 24 of the preceding clip 12 therebetween.

The length of the second region 34 is substantially equal to the movement length required for the clamping portion 40 set at the initial position with respect to the clip 12 to move until the clamping of the clip 12 is completed. That is, while the clip 12 retreats relative to the connection ring 14 to be clamped, the second region 34 of the connection ring 14 maintains the connection between the two clips 12 retained therein, allowing the pulling force of the rear clip 12 to be transmitted to the front clip 12, and when the clamping has been completed, the engagement portion of the two clips 12 is detached from the second region 34, thereby canceling the connection between the clips 12.

As illustrated in FIG. 3C, the second region 34 has a hole 43 having the same inner diameter as the proximal end side portion of the first region 32, and further, two grooves (recesses) 43a opposed to each other are formed. The grooves 43a can accommodate the arm portions 28 of the clip 12 retained in the second region 34, with the claw portions 22 being closed.

The grooves 43a are provided at two positions in the direction in which the claw portions 22 of the clip 12 retained in the second region 34 are opened and closed (horizontal direction in FIGS. 3B and 3C). The plate surfaces of the arm portions 28 of the clip 12 retained in the second region 34 abut the inner walls of the grooves 43a. The width (opening width) of the grooves 43a is slightly larger than the maximum width of the arm portions 28 of the clip 12, and the distance from the wall surface of one groove 43a to the wall surface of the other groove 43a is substantially equal to the sum total of the lengths of the two claw portions 22 of the clip 12 (length in the diverging direction). The width of the grooves 43a is smaller than the width of the projections 30 formed on the arm portions 28. Thus, the projections 30 of the clip 12 retained in the second region 34 cannot enter the grooves 43a.

The distance between the wall surfaces of the two grooves 43a is such that the engagement between the turned portion 24 of the preceding clip 12 and the claw portions 22 of the next clip 12 is not canceled, and the distance is smaller than the sum total of the lengths of the two claw portions 22 and the width of the portion of the turned portion 24 engaged with the claw portions 22.

For example, the claw portions 22 of the clip 12 retained in the second region 34 may slightly overlap each other, or the connection of the clip with the preceding clip 12 may be maintained, with a slight gap being left between the claw portions 22.

The engagement portion between the two clips 12 is retained in the portion of the second region 34 close to the boundary between the second region 34 and the first region 32. Inside the sheath 16, the turned portion 24 of the preceding clip 12 (e.g., the clip 12B in the connection ring 14B illustrated in FIG. 1B) is retained by the closed skirt portions 38 in the first region 32, and hence the advancing/retreating movement and rotating movement of the clip is restrained. The next clip 12 (e.g., the clip 12C in the connection ring 14B illustrated in FIG. 1B) engaged with the preceding clip 12 is retained in an orientation differing by 90 degrees from the preceding clip by the grooves 43a of the second region 34, whereby rotating movement of the clip is restrained, and the clip is engaged with the preceding clip restrained in advancing/retreating movement, thereby restraining the advancing/retreating movement thereof. That is, the engagement portion between the front and rear clips is retained by the connection ring 14 with very little play.

As illustrated in FIGS. 1A and 1B, the claw portions 22 of the second clip 12B are engaged with the turned portion 24 of the first clip 12A, and the engagement portion is retained by the connection ring 14A. The claw portions 22 of the second clip 12B are retained in the closed state by the inner wall of the connection ring 14A (second region 34 thereof). As a result, the connection of the first clip 12A and the second clip 12B is maintained. Similarly, the connection of the second clip 12B and the third clip 12C is maintained by the connection ring 14B, the connection of the third clip 12C and the fourth clip 12D is maintained by the connection ring 14C, and the connection of the fourth clip 12D and the dummy clip 18 is maintained by the connection ring 14D.

The rearmost clip 12D is engaged with the dummy clip 18, which is not used for clipping. The dummy clip 18 is a member having a resilient portion of a configuration similar to that of the open end side half as from the crossing portion 26 of the clip 12. The resilient portion is engaged with the turned portion of the clip 12D, with the claw portions thereof being closed, and releases the clip 12D when the claw portions are opened. The manipulating wire 20 is fixedly connected to the proximal end portion of the dummy clip 18.

The sheath 16 is formed, for example, of a coil sheath formed through intimate winding of metal wire. The inner diameter of the sheath 16 is one allowing canceling of the engagement between the turned portion 24 of the preceding clip 12 and the claw portions 22 of the next clip 12. That is, the inner diameter of the sheath 16 is larger than the sum total of the lengths of the two claw portions 22 and the width of the turned portion 24 engaged with the claw portions 22.

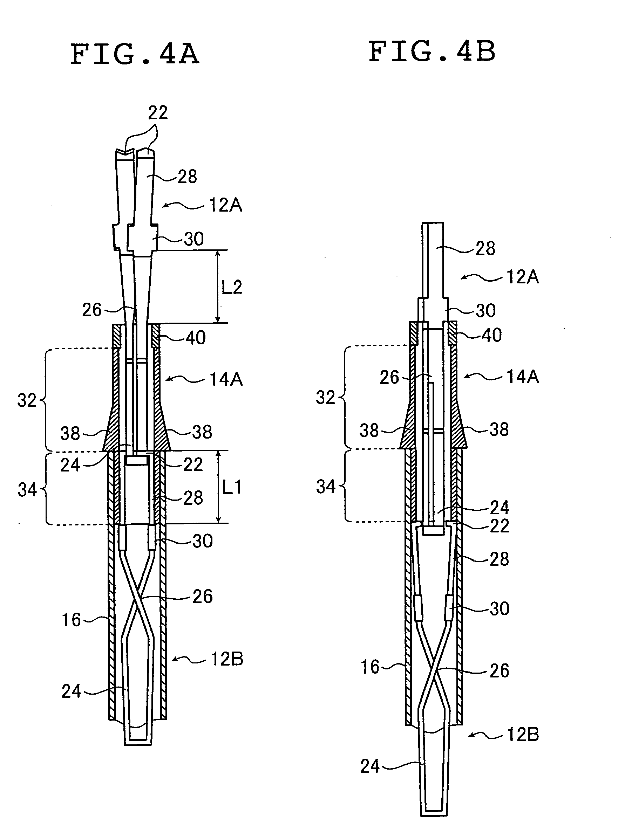

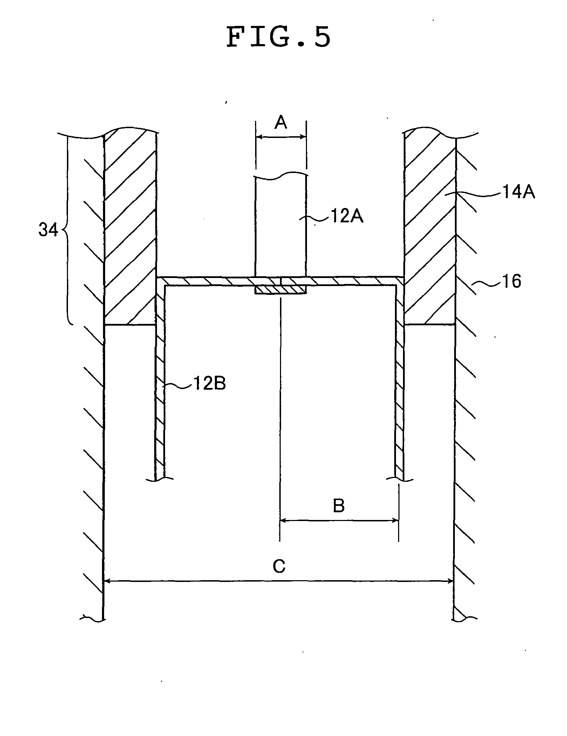

Here, the construction of the connection ring 14 and the movement of each portion of the clip 12 and the connection ring 14 during clipping operation are described in detail with reference to FIGS. 4A, 4B, and 5. FIGS. 4A and 4B are partial sectional views illustrating stepwise the condition of the clips 12A and 12B and the connection ring 14A during the clipping operation by the foremost clip 12A. In those drawings, the connection ring 14B for retaining the clip 12B is omitted. FIG. 5 is an enlarged view illustrating the proximal end portion of the connection ring 14A and the engagement portion between the clips 12A and 12B in the sheath 16.

FIG. 4A illustrates an initial state (standby state) in which the clip 12A can be used. In the state of FIG. 4A, the region length L1 of the second region 34, which is a connection maintaining region, and the length L2 measured from the lower end of the projections 30 of the clip 12A to the upper end of the connection ring 14A are substantially equal to each other. With the claw portions 22 being closed with the tail portion of the clip 12A therebetween, the arm portions 28 of the clip 12B are retained by the second region 34.

While the clip 12A and the clip 12B are retained by the connection ring 14A in the connected state, the skirt portions 38 of the connection ring 14A are already out of the forward end of the sheath 16 and open, and hence the pressurizing retention of the clip 12A by the skirt portions 38 is canceled. Since the skirt portions 38 are open at the forward end of the sheath 16, the connection ring 14A is prevented from retreating into the sheath 16.

When, in this state, the clip 12B is pulled by pulling the manipulating wire 20 (see FIGS. 1A and 1B), the clip 12A is pulled by the claw portions 22 of the clip 12B. By pulling the manipulating wire 20 by a predetermined amount, the clip 12A moves by the length L2 with respect to the connection ring 14A, and, as illustrated in FIG. 4B, the lower ends of the projections 30 of the clip 12A reach the position where they abut the forward end of the connection ring 14A or a position immediately above the same. As a result, the clamping of the clip 12A by the clamping portion 40 of the connection ring 14A is completed.

At this time, the clip 12B also moves by the same amount as the clip 12A. That is, it moves by the region length L1 of the second region 34, which is substantially equal to the above-mentioned length L2, and the forward end of the clip 12B leaves the proximal end of the connection ring 14A, with the engagement portion between the clip 12A and the clip 12B being detached from the second region 34 of the connection ring 14A.

As stated above, the inner diameter of the sheath 16 is larger than the sum total of the lengths of the two claw portions 22 of the clip 12B and the width of the portion of the turned portion 24 engaged with the claw portions 22, and hence the clip 12B detached from the connection ring 14A is diverged until the arm portions 28 thereof abut the inner wall of the sheath 16 to thereby form between the claw portions 22 a gap allowing passage of the lower end portion of the clip 12A.

FIG. 5 is an enlarged sectional view of the lower end portion of the connection ring 14A in the state immediately before detachment of the clip 12B from the connection ring 14A. Assuming that the width of the portion of the clip 12A engaged with the clip 12B is A, that the length of the claw portions 22 of the clip 12B is B, and that the inner diameter of the sheath 16 is C, the following relationship holds good: C > A + B × 2. For example, when A = 0.3 mm, B = 0.7 mm, and C = 2.2 mm, the arm portions 28 of the clip 12B, which has left the second region 34 of the connection ring 14A, are diverged, and there is formed between the claw portions 22 a gap of 2.2 mm - 0.7 mm × 2 = 0.8 mm allowing passage of the clip 12A having a width of 0.3 mm.

As a result, the connection between the clip 12A and the clip 12B is canceled, and the clip 12A and the connection ring 14A can be detached from the sheath 16, whereby the clipping by the clip 12A and the connection ring 14A is completed.

Next, the clipping operation of the magazine type clipping device 10 is described. FIGS. 6A through 6E are partial sectional views illustrating stepwise the condition of the clipping device 10 during clipping manipulation.

First, as illustrated in FIG. 6A, the sheath 16 is loaded with four bleeding stop clip members including the clips 12A through 12D and the connection rings 14A through 14D, and then the sheath 16 is inserted into the forceps channel of the endoscope. The loading of the bleeding stop clip members can be effected, for example, as follows: the four bleeding stop clip members (that are obtained by fitting the connection rings 14 onto the clips 12) and the dummy clip 18 are connected together beforehand, and the dummy clip 18 is attached to the forward end of the manipulating wire 20 protruding from the forward end of the sheath 16; after this, the sheath 16 is caused to advance with respect to the manipulating wire 20, and the foremost clip 12A is completely accommodated in the sheath 16.

As illustrated in FIG. 6A, the forward end of the clip 12A is substantially matched with the forward end of the sheath 16. The foremost clip 12A is maintained in the closed state by the inner wall of the sheath 16. The connection rings 14A through 14D are fitted such that, in the initial state, the clamping portions 40 thereof are in the vicinity of the crossing portions 26 of the clips 12A through 12D. At this time, the upper ends of the projections 30 of the clips 12B through 12D as seen in FIG. 6A are respectively situated directly below the connection rings 14A through 14C.

When the forward end of the sheath 16 reaches the forward end of the insert portion of the endoscope inserted into the living body, and protrudes from the forward end of the endoscope, solely the sheath 16 is pulled to the manipulating portion side, with the manipulating wire 20 remaining as it is. When the sheath 16 is pulled by a predetermined stroke, the forward end of the sheath 16 is lowered to a position where the skirt portions 38 of the foremost connection ring 14A are opened, and the claw portions 22 of the clip 12A protruding from the sheath 16 are expanded by the urging force, resulting in the state of FIG. 6B. As a result, the first clip 12A is usable. In FIG. 6B, the skirt portions 38 of the connection ring 14A are perpendicular to the plane of the drawing, and hence they are not illustrated.

The connecting portion between the clip 12A and the clip 12B is situated directly below the skirt portions 38 of the connection ring 14A, and hence, in the state of FIG. 6B, the forward end of the clip 12B substantially coincides with the forward end of the sheath 16. That is, the length of one stroke by which the sheath 16 is drawn is substantially equal to the distance between the forward end of the clip 12A loaded into the sheath 16 and the forward end of the clip 12B.

When the sheath 16 is drawn, there is exerted a frictional force between the sheath 16 and the connection rings 14A through 14D fitted into the sheath 16. In addition to the elastic force of the skirt portions 38 and the retaining force due to the resilient force of the clips 12A through 12D exerted between the connection rings 14A through 14D and the clips 12A through 12D, the projections 30 of the clips 12B through 12D abut the proximal ends of the connection rings 14A through 14C, which means the projections cannot enter the holes 43 of the connection rings 14, and hence, even if the sheath 16 is drawn, the connection rings 14A through 14D make no unnecessary movement. Thus, the connection rings 14A through 14D can maintain the state of retaining the clips 12A through 12D.

Next, the clipping device 10 in the state of FIG. 6B is moved to press the claw portions 22 of the diverged clip 12A against the portion to be subjected to clipping, and the manipulating wire 20 is pulled by a predetermined amount on the proximal end side of the sheath 16. By pulling the manipulating wire 20, the clips 12A through 12D engaged sequentially starting from the dummy clip 18 are pulled all together.

At this time, in the state of FIGS. 6B and 6C, the skirt portions 38 of the connection ring 14A protruding from the forward end of the sheath 16 are open, and the retention of the clip 12A by the connection ring 14A is released. Thus, as illustrated in FIG. 6C, the foremost clip 12A retreats relative to the connection ring 14A. The forward end of the connection ring 14A, that is, the clamping portion 40, is pushed down to a position directly below the projections 30 of the clip 12A, whereby the clamping of the clip 12A by the connection ring 14A is completed.

At the same time, the engagement portion between the clip 12A and the next clip 12B leaves the rear end of the connection ring 14A. When the engagement portion between the clip 12A and the clip 12B is detached from the connection ring 14A, the arm portions 28 are diverged by the resilient force of the clip 12B until they abut the inner wall of the sheath 16, and the claw portions 22 are opened until their interval becomes larger than the width of the turned portion 24 of the clip 12A, thereby canceling the connection between the clip 12A and the clip 12B. As a result, the foremost clip 12A becomes detachable, and the clipping by the clip 12A is completed.

On the other hand, the succeeding clips 12B through 12D are retained by the connection rings 14B through 14D whose skirt portions 38 are closed so as not to move in the rotating direction and the advancing/retreating direction with respect to the connection rings 14B through 14D. Further, the claw portions 22 are pressed against the inner walls of the second regions 34 of the connection rings 14B through 14D by the expanding force (urging force) of the claw portions 22 of the clips 12C and 12D engaged with the clips 12B through 12D and the claw portions of the dummy clip 18, with the result that the frictional force between the clips 12B through 12D and the connection rings 14B through 14D is enhanced. Thus, the connection rings 14B through 14D move with the movement of the clips 12B trough 12D. That is, the clips and the connection rings other than the foremost clip 12 and the connection ring 14 retaining the same advance and retreat integrally with respect to the sheath 16, and the connected state of the clips 12B through 12D and the dummy clip 18 is maintained by the connection rings 14B through 14D.

The manipulating wire 20 is formed so as to be always capable of being pulled by a fixed amount. This fixed amount is equal to the region length L1 of the second region 34 of the connection ring 14 illustrated in FIG. 4A, that is, the length L2 measured from the lower ends of the projections 30 of the preceding clip 12 to the upper end of the connection ring 14, or somewhat larger than that. Further, the manipulating wire 20 is adapted to be quickly returned by the fixed amount after being pulled by the fixed amount. When the pulling force is released at the manipulating portion, the manipulating wire 20 pulled from the state of FIG. 6B to the state of 6C is restored to the former position, resulting in the state of FIG. 6D. That is, as in the state of FIG. 6B, the forward end of the second clip 12B returns to a position where it is substantially matched with the forward end of the sheath 16.

Next, in order to place the second clip 12B in the usable state, the sheath 16 is pulled by the predetermined one stroke. As a result, the forward end of the sheath 16 is lowered to the position where the skirt portions 38 of the next connection ring 14B are opened, and the claw portions 22 of the clip 12B protruding from the sheath 16 are diverged, whereby the state as illustrated in FIG. 6E is attained.

After that, as in the case of the clip 12A described above, the claw portions of the clip 12B are pressed against the portion to be subjected to clipping, and the manipulating wire 20 is pulled by a predetermined amount. As a result, the clamping of the clip 12B by the connection ring 14B is completed, and, at the same time, the connection between the clip 12B and clip 12C is canceled, whereby the clipping by the clip 12B is completed.

As described above, in the clipping device 10 of Embodiment 1, the connection rings 14 cover the connecting portions between the clips 12 to retain the same, and hence the plurality of clips 12 are reliably maintained in the connected state. Further, by pulling the dummy clip 18 and the plurality of clips 12 connected thereto by a predetermined length by the manipulating wire 20, it is possible to simultaneously effect the clamping of the foremost clip 12 by the clamping portion 40 of the connection ring 14 and the canceling of the connection with the next clip, thus effecting the clipping by the foremost clip 12.

Further, by pulling back the sheath 16 to the manipulating portion side by a predetermined length, the next clip 12 can be used, thus making it possible to successively perform clipping.

Further, since the connecting portions between the clips 12 are covered with the connection rings 14, there is no fear of the inner wall of the sheath 16 being damaged by the corner portions or the like of the clips 12 at the time of clipping operation, etc. Further, when the sheath 16 is inserted into the endoscope 16, there is very little possibility of twisting or distortion being generated in the clips 12 at the connecting portions.

Further, in the clipping device 10 of Embodiment 1, as a preferred mode, the connection ring 14 is formed of resin, and hence the friction between the connection ring 14 and the inner wall of the sheath 16 is small, and it is possible to smoothly perform the manipulation of causing the clip 12 to advance and retreat by the manipulating wire 20 and the manipulation of pulling the sheath 16, with there being no fear of the inner wall of the sheath 16 being flawed.

The sheath 16 loaded with the clips 12 has to pass a curved portion in the endoscope when being inserted into the endoscope inserted into the living body. In this regard, when the connection ring 14 is formed of resin, the connection ring 14 is superior in flexibility, and can be bent while retaining the connecting portion of the clips 12.

In the clipping device 10 of Embodiment 1, in the state in which the connection ring 14 is set in the sheath 16, the skirt portions 38 of the connection rings 14 retain the clips 12 through pressurization, and hence it is possible to retain the connecting portions of the clips 12 in a fixed state, and there is very little play in the connecting portions. Thus, the advancing/retreating movement at the time of manipulation by the manipulating wire 20 is stabilized, and the error in the movement amount is small, making it possible to effect movement with high precision.

While in the above-mentioned example the clips 12 are connected together with their orientations alternately changed by 90 degrees, this should not be construed restrictively, and the inner configuration of the connection clip can be selected according to the configuration of the engagement portion. For example, it is also possible to adopt a clip of a configuration in which twisting is effected by 90 degrees at the portion between the claw portions 22 and the turned portion 24, connecting together the consecutive clips in the same orientation. Further, by using a closed clip with a turned portion, it is advantageously possible to impart a resilient force (urgent force) pressurizing the turned portion and diverging the arm portions. The present invention, however, is also applicable to a construction adopting an open clip (U-shaped clip) with no turned portion.

### Embodiment 2

A magazine type clipping device according to Embodiment 2 of the present invention is described. Embodiment 2 differs from Embodiment 1 described above in the construction of the connection ring. Otherwise, it is basically of the same component construction and operation as Embodiment 2.

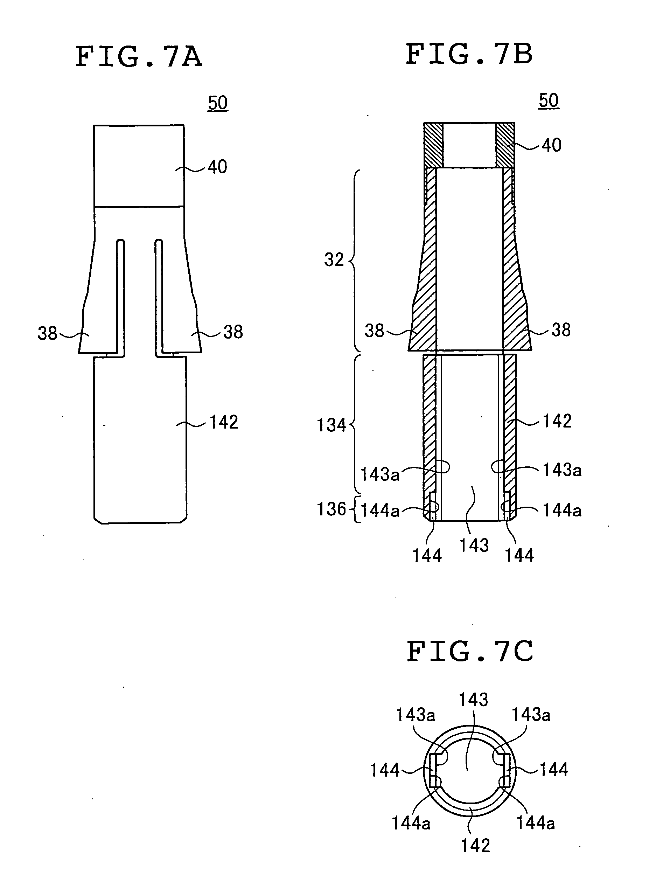

FIGS. 7A through 7C are a front view, a sectional view, and a bottom view of a connection ring 50 according to Embodiment 2. The connection ring 50 is of the same structure as the connection ring 14 of Embodiment 1 except that a retaining portion 142 has a third region 136 continuous with a second region 134. That is, the connection ring 50 is composed of the metal clamping portion 40 and the resin retaining portion 142, and the retaining portion 142 has the first region 32 retaining the preceding clip 12, the second region 134, which is a connection maintaining region retaining the succeeding clip 12 connected to the preceding clip 12, and the third region 136, which is a connection canceling region allowing canceling of the connection of the two consecutive clips 12.

The third region 136 is provided on the proximal end side of the second region 134, that is, at the proximal end portion of the connection ring 50. As illustrated in FIGS. 7B and 7C, the third region 136 has a hole continuous with and of the same diameter as a hole 143 of the second region 134, and grooves 144 situated at the same positions as grooves 143a of the second region 134 and of a larger depth.

The distance between the wall surfaces of the grooves 143a of the second region 134 is substantially equal to the sum total of the lengths (lengths in the diverging direction) of the two claw portions 22 of the clip 12, and the grooves accommodate the arm portions 28 of the clip 12 in the closed state with the claw portions 22 thereof holding therebetween the closed end (tail portion) of the turned portion 24 of the preceding clip 12. As a result, the second region 134 maintains the two consecutive clips 12 in the connected state.

In contrast, the distance between the opposing inner walls 144a of the grooves 144 of the third region 136 is slightly larger than the sum total of the length of the two claw portions 22 of the clip 12 and the width of the portion (tail portion) of the turned portion 24 engaged with the claw portions 22, and the two claw portions 22 of the clip 12 are opened to provide between the claw portions 22 a distance allowing detachment of the tail portion of the preceding clip 12.

On the other hand, the width of the grooves 144 (opening width) is the same as the width of the grooves 143a of the second region 134. That is, like the width of the grooves 143a, the width of the grooves 144 is slightly larger than themaximumwidth of the arm portions 28 of the clip 12 but smaller than the width of the projections 30 formed on the arm portions 28, and hence the projections 30 of the clip 12 retained by the second region 134 cannot enter the grooves 144.

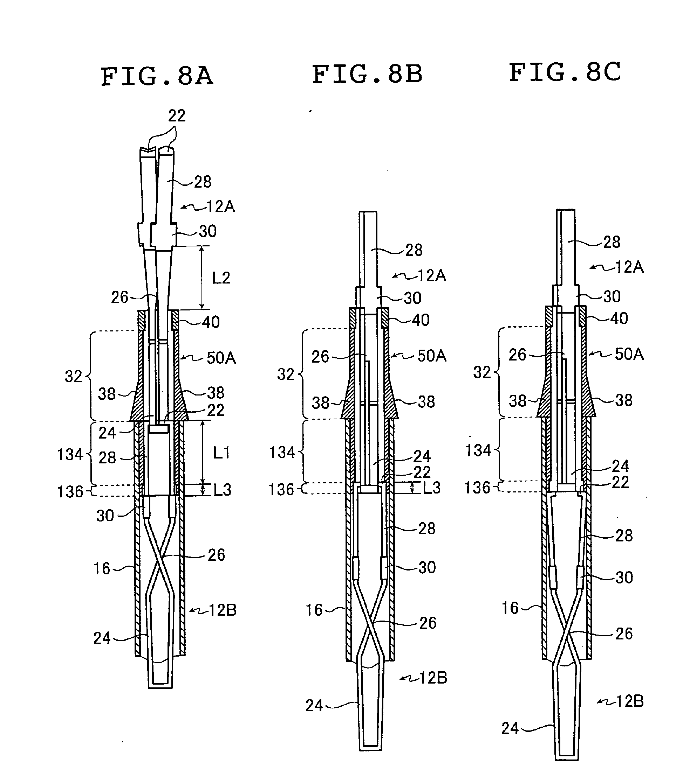

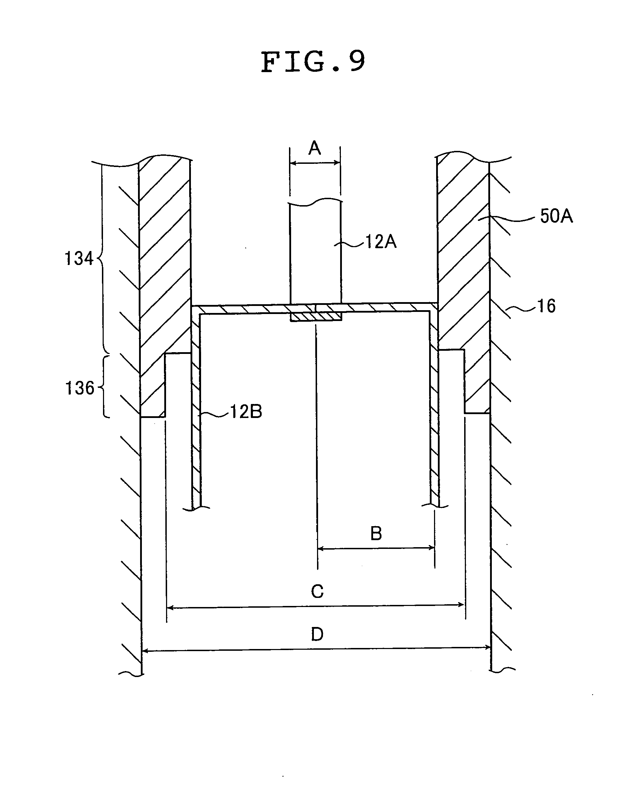

FIGS. 8A through 8C are partial sectional views illustrating stepwise the condition of the clips 12A and 12B and the connection ring 50A during the clipping operation by the foremost clip 12A. In the drawings, the connection ring 50B retaining the clip 12B is omitted. FIG. 9 is an enlarged view of the proximal end portion of the connection ring 50A and the engagement portion between the clip 12A and the clip 12B in the sheath 16.

FIG. 8A illustrates an initial state (standby state) in which the clip 12A is usable. The skirt portions 38 of the connection ring 50A are diverged at the forward end of the sheath 16 to cancel the pressurizing retention of the clip 12A, and, at the same time, prevents the connection ring 50A from retreating into the sheath 16. In the state of FIG. 8A, the region length L1 of the second region 134, which is a connection maintaining region, and the length L2 from the lower end of the projections 30 of the clip 12A to the upper end of the connection ring 50 are substantially equal to each other.

When, in this state, the clip 12B is pulled by pulling the manipulating wire, the clip 12A is pulled by the claw portions 22 of the clip 12B. By pulling the manipulating wire by a predetermined amount, the clip 12A moves by the length L2 with respect to the connection ring 50A, and, as illustrated in FIG. 8B, the lower ends of the projections 30 of the clip 12A reach the position where they abut the forward end of the connection ring 50A or a position immediately above the same. As a result, the clamping of the clip 12A by the clamping portion 40 of the connection ring 50 is completed.

At this time, the clip 12B moves by the same amount as the clip 12A. That is, it moves by the length L1 of the second region 134, which is substantially equal to the above length L2, and the forward end of the clip 12B leaves the second region 134 of the connection ring 50A, with the engagement portion between the clip 12A and the clip 12B being detached from the second region 134 to enter the third region 136.

As stated above, the grooves 144 formed in the third region 136 are deeper than the grooves 143a formed in the hole 143 of the second region 134 in the diverging direction of the clip 12B, and the distance between the inner walls 144a of the two grooves 144 is slightly larger than the sum total of the length of the two claw portions 22 of the clip 12B and the width of the portion of the turned portion 24 engaged with the claw portions 22, and hence arm portions 28 of the clip 12B moved to the third region 136 are diverged, providing between the claw portions 22 a distance allowing passage of the lower end portion of the clip 12A. That is, the clip 12A and the clip 12B are disengaged with each other, and are placed in a state in which their connection can be canceled.

FIG. 9 is an enlarged sectional view of the lower end portion of the connection ring 50 immediately before detachment of the clip 12B from the connection ring 50. As illustrated in FIG. 9, assuming that the width of a portion of the clip 12A engaged with the clip 12B is A, that the length of the claw portions 22 of the clip 12B is B, and that the distance between the wall surfaces of the grooves 144 of the third region 136 is C, the following relationship holds good: C ≥ A + B × 2.

For example, when A = 0.3 mm, B = 0.7 mm, and C = 1.8 mm, the arm portions 28 of the clip 12B which has left the second region 134 of the connection ring 50A and entered the third region 136 thereof are diverged, providing between the claw portions 22 an interval of 1.8 mm - 0.7 mm × 2 = 0.4 mm allowing passage of the tail portion of the clip 12A having a width of 0.3 mm.

However, the distance between the inner walls 144a of the grooves 144 of the third region 136 illustrated in FIGS. 7B and 7C is substantially smaller than the natural diverging amount of the claw portions 22 of the clip 12, and hence a pressurization force due to the resilient force (urgent force) of the clip 12B is exerted on the inner walls 144a of the grooves 144, and a frictional force is exerted between the arm portions 28 of the clip 12B retained in the third region 136 and the inner walls 144a of the grooves 144 of the third region 136. As a result, as illustrated in FIG. 8B, at the point in time when the engagement portion between the clip 12A and the clip 12B has left the second region 134 and entered the third region 136, the clip 12A and the clip 12B are disengaged from each other. However, the succeeding clip 12B is not immediately detached from the connection ring 50, and there is maintained a state in which the two clips 12A and 12B are both retained by the single connection ring 50A. Thus, the connection ring 50A and the clip 12A retained by the connection ring 50A still remain attached to the sheath 16.

When the manipulating wire is further pulled, and the clip 12B is pulled, solely the clip 12B moves by the region length L3 of the third region 136, and, as illustrated in FIG. 8C, the forward end of the clip 12B is detached from the third region 136 of the connection ring 50A, thus being detached from connection ring 50A.

Here, the inner diameter of the sheath 16 is larger than the distance between the inner walls 144a of the grooves 144 of the third region 136 of the connection ring 50, and larger than the sum total of the length of the two claw portions 22 and the width of the portion of the turned portion 24 engaged with the claw portions 22. Thus, when, in the sheath 16, the proximal end side clip 12 is diverged, there is generated between the claw portions 22 thereof an interval large enough to cause the turned portion 24 of the forward end side clip 12 to be detached.

In FIG. 9, assuming that the inner diameter of the sheath 16 is D, the inner diameter D, the width A of the portion of the clip 12A engaged with the clip 12B, the length B of the claw portions 22 of the clip 12B, and the inter-wall-surface distance C of the grooves 144 of the third region 136 are in the following relationship: D > C, and D > A + B × 2.

For example, when D = 2.2 mm, there is generated between the claw portions 22 of the clip 12B, which has been detached from the third region 136 of the connection ring 50A and diverged up to the inner diameter of the sheath 16, an interval of 2.2 mm - 0.7 mm × 2 = 0.8 mm allowing the clip 12A of the width of 0.3 mm to pass without a hitch. Due to the detachment of the clip 12B from the connection ring 50A, the frictional force between the clip 12B and the connection ring 50A ceases to be exerted.

As a result, the clip 12A and the connection ring 50A can be detached from the sheath, and the clipping operation by the clip 12A and the connection ring 50A is completed.

In this way, the connection ring 50 has the third region 136, which is a connection canceling region, and hence, if the clip 12 retained within the sheath 16 is deviated with respect to the connection ring 50 due to curving of the sheath 16 or for some other reason, and the connecting portion of the clip 12 is detached to the proximal end side from the second region 134, which is a connection maintaining region of the connection ring 50, the clip 12 is not easily detached from the connection ring 50, and it is possible to prevent the clip 12 from being immediately disconnected within the sheath 16 to drop from the forward end of the sheath 16.

In Embodiment 2, the manipulating wire pulling amount for one clipping operation is equal to or larger than the region length (L1) of the second region 134 + the region length (L3) of the third region 136. L1, or L2, is the length necessary for the clamping of the clip 12A, and L3 is the length necessary for the canceling of the connection of the clips 12. The pulling of the manipulating wire can be effected through a single continuous pulling operation. It is also possible for the series of pulling operations for the manipulating wire to be intermittently performed in two steps of L1 and L3.

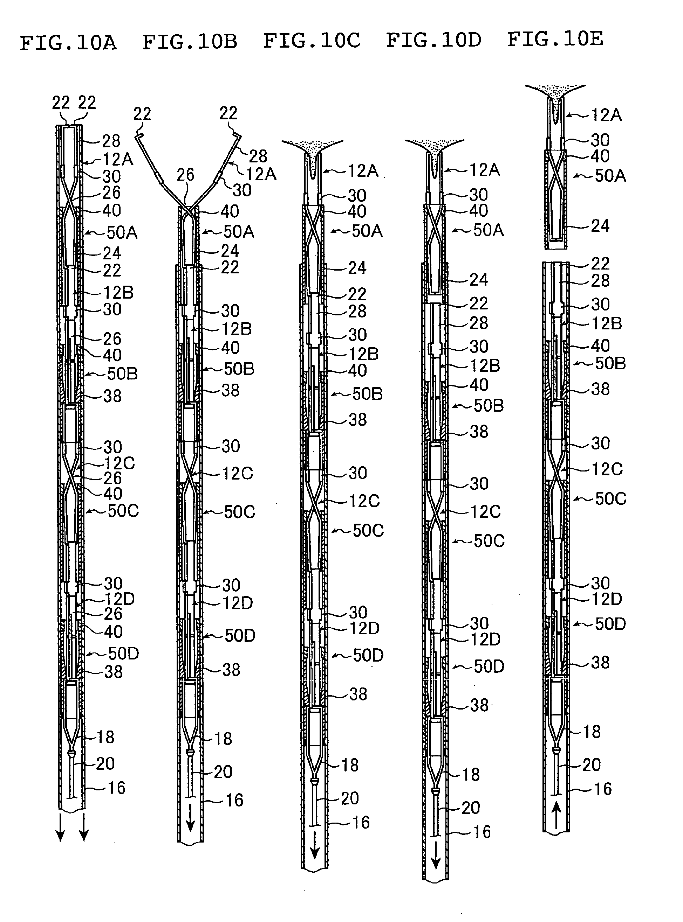

FIGS. 10A through 10E are partial sectional views illustrating stepwise the condition of the clipping device of Embodiment 2 during clipping operation. In the following, the operation of the clipping device of Embodiment 2 is described, concentrating on how it differs from that of Embodiment 1.

As illustrated in FIG. 10A, in the state in which the sheath 16 is loaded with four bleeding stop units consisting of the clips 12A through 12D and the connection rings 50A through 50D, the engagement portions of the clips 12A through 12D are retained by the second region 134, which is the connection maintaining region for the connection ring 50A.

When, with the manipulating wire 20 remaining as it is, solely the sheath 16 is pulled to the manipulating portion side by a predetermined stroke, the skirt portions 38 of the connection ring 50A of the foremost ring 50A are opened, resulting in the state of FIG. 10B. As a result, the first clip 12A becomes usable. In FIG. 10B, the skirt portions 38 of the connection ring 50A are perpendicular to the plane of the drawing, and hence they are not illustrated in the drawing.

Next, when the claw portions 22 of the diverged clip 12A are pressed against the portion to be subjected to clipping, and the manipulating wire 20 is pulled by a predetermined amount on the proximal end side of the sheath 16, all the clips 12A through 12D, successively engaged with each other starting from the dummy clip 18, are pulled together.

In the state of FIG. 10B, the skirt portions 38 of the connection ring 50A at the forward end of the sheath 16 are open, and the retention of the clip 12A by the connection ring 50A is canceled, and hence, as illustrated in FIG. 10C, by pulling the manipulating wire 20, the foremost clip 12A retreats with respect to the connection ring 50A. The forward end of the connection ring 50A, that is, the clamping portion 40, is forced into a position directly below the projections 30 of the clip 12A, whereby the clamping of the clip 12A by the connection ring 14A is completed.

At the same time, the engagement portion between the clip 12A and the clip 12B leaves the second region 134 of the connection ring 50A, and moves to the third region 136, which is a connection canceling region. When the engagement portion between the clip 12A and the clip 12B moves to the third region 136, the arm portions 28 are diverged by the urging force of the clip 12B until they abut the inner walls 144a of the grooves 144, and the interval between the claw portions 22 of the clip 12A becomes slightly larger than the width of the turned portion 24 of the clip 12A, resulting in a state in which the connection between the clip 12A and the clip 12B can be canceled.

However, in the state in which the clip 12B is in the third region 136, there is exerted a frictional force between the connection ring 50A retaining the clip 12A and the next clip 12B, and hence the connection ring 50A is not detached from the clipping device (sheath 16).

When the manipulating wire 20 is further pulled, solely the clip 12B retreats because the engagement between the clip 12A and the clip 12B has already been canceled. As illustrated in FIG. 10D, when the clip 12B is moved until it leaves the rear end of the connection ring 50A, the foremost clip 12A and the connection ring 50A can be detached from the sheath 16, and, by further pulling forward the entire clipping device, the foremost clip is completely separated as illustrated in FIG. 10E. As a result, the clipping operation by the clip 12A and the connection ring 50A is completed.

The manipulating wire 20 can be pulled by a fixed amount which is equal to or somewhat larger than the sum total of the region length L1 of the second region 134 of the connection ring 50 and the region length L3 of the third region 136 illustrated in FIG. 8A. When, after the manipulating wire 20 has been pulled by this fixed amount, the pulling force is canceled, the manipulating wire 20 moves in the opposite direction by this fixed amount to return to the former position. When the pulling force is canceled after the manipulating wire 20 has been pulled by a series of operations in one direction from the state of FIG. 10B to that of FIG. 10C and from the state of FIG. 10C to that of FIG. 10D, the manipulating wire 20 returns to the position of FIG. 10E, that is, the same position as that of FIG. 10B.

In this way, in addition to the construction of Embodiment 1, in Embodiment 2, the connection ring 50 is provided with the connection canceling region continuous with the connection maintaining region maintaining the connected state, whereby, if the connecting portion of the clip 12 should be detached from the connection maintaining region (second region 134) of the clip 12 within the sheath 16, the succeeding clip 12 retained by the connection ring 50 is kept retained in the connection canceling region (third region 136) of the connection ring 50, and hence it is possible to prevent the clip 12 from being immediately disconnected within the sheath 16 to drop from the forward end of the sheath 16.

### Embodiment 3

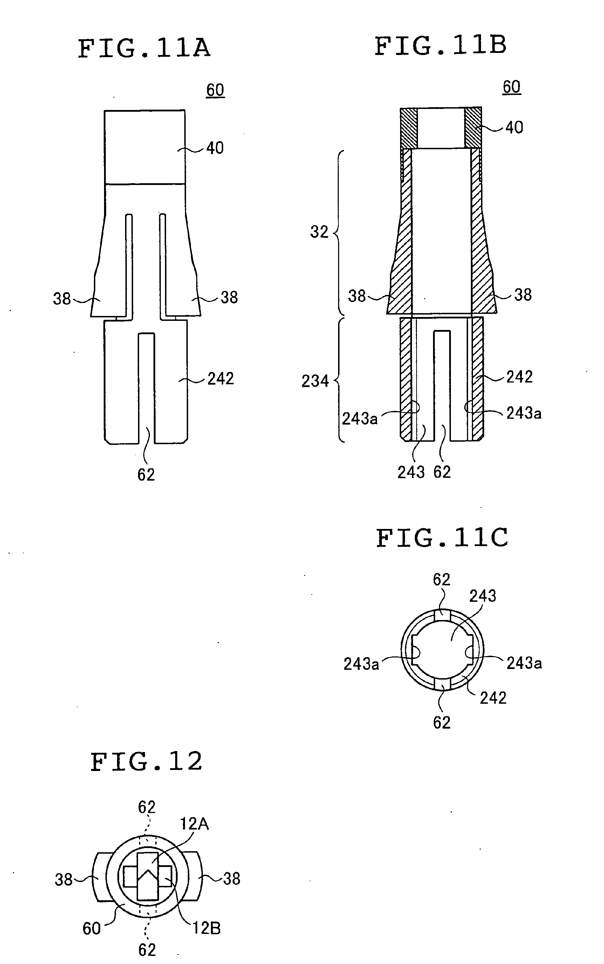

Instead of the connection rings 14 of the clipping devices 10 of Embodiment 1, it is possible to use a connection ring 60 as illustrated in FIGS. 11A through 11C.

The connection ring 60 is of the same construction as the connection ring 14 of Embodiment 1 except that two slits 62 are formed in a second region 234 of a retaining portion 242. That is, the connection ring 60 includes the metal clamping portion 40 and the resin retaining portion 242, and the retaining portion 242 has a first region 32 and a second region 234, with the second region 234 having the slits 62 cut from the proximal end thereof at positions opposed to each other.

The slits 62 are cutouts of a certain width open on the proximal end side of the connection ring 60 and substantially parallel to the axis of the connection ring 60. It is desirable for the slits 62 to be provided at circumferential positions of the connection ring 60 different fromthose of the skirt portions 38. Further, it is desirable for the slits 62 to be formed shallow enough not to reach the skirt portions 38. With this configuration, it is possible to prevent the strength of the connection ring 60 from being substantially reduced.

Further, it is desirable for the slits 62 to be provided at positions different from those of grooves 243a of the second region 234, that is, positions different from the diverging direction of the clips 12 accommodated in the second region 234. Further, it is desirable for the slits 62 to be formed shallow enough not to reach the position of the rear end of the clip 12 retained by the first region 32, that is, the engagement position between the clips 12. Due to this configuration, even in a connection clip unit not yet loaded into the sheath 16, it is possible to maintain the retention of the clips 12 in the second regions 234 of the connection rings 62.

The depth of the slits 62 is set within a range satisfying the above requirement according to the minimum curving radius required of the clipping device 10.

In the connection ring 60 illustrated in FIGS. 11A through 11C, two slits 62 are formed at positions deviated from the skirt portions 38 by 90 degrees to extend to a position shallow enough not to reach the upper end of the second region 234. As in the illustrated example, the closed ends (upper ends as seen in the drawing) of the slits 62 may be rectangular, or of a round, semi-circular configuration.

FIG. 12 is a diagram schematically illustrating the positional relationship between the skirt portions 38 of the connection ring 62 and the clips 12A and 12B when the connection ring 62 is retaining the clip 12A and the clip 12B. In order to indicate the position of the slits 62, the clip 12A is illustrated as closed.

Due to the provision of the above-mentioned slits 62, the connection ring 60 is improved in terms of flexibility, and the clipping device 10 can pass a curved portion of small curvature. Further, due to the provision of the slits 62, the hem (proximal end portion) of the connection ring 60 is partially turned up, and hence, when the front and rear clips 12 are connected together prior to the loading of the clips 12 into the sheath 16, the connection is advantageously facilitated through the turning of the hem of the connection ring 60.

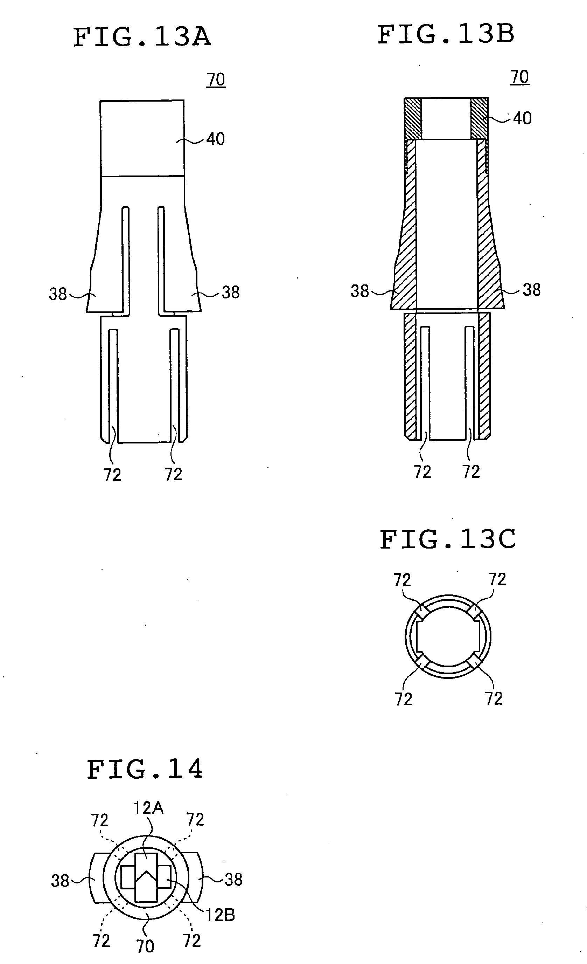

As illustrated in FIGS. 13A through 13C and FIG. 14, it is also possible to use a connection ring 70 having four slits 72. The four slits 72 are provided at positions deviated by 45 degrees from the central positions of the two skirt portions 38. It is also possible to provide the slits at positions deviated therefrom by some other angle, e.g., 60 degrees.

While it is desirable to provide two or more slits from the viewpoint of achieving an improvement in terms of flexibility and assembly property, it is also possible to provide only one slit. If there is provided only one slit, it is possible to achieve an improvement in terms of flexibility. When there are provided two or more slits, it is desirable to provided them in an even number, and it is desirable to form them to be arranged at positions symmetrical with respect to the positions of the skirt portions 38, that is, at positions at an equal distance from the skirt portions 38. However, when too many slits are provided, it is impossible to maintain the requisite strength for the connection ring, and hence, when setting the number and configuration of the slits, it is necessary to take the strength of the connection ring into consideration.

### Embodiment 4

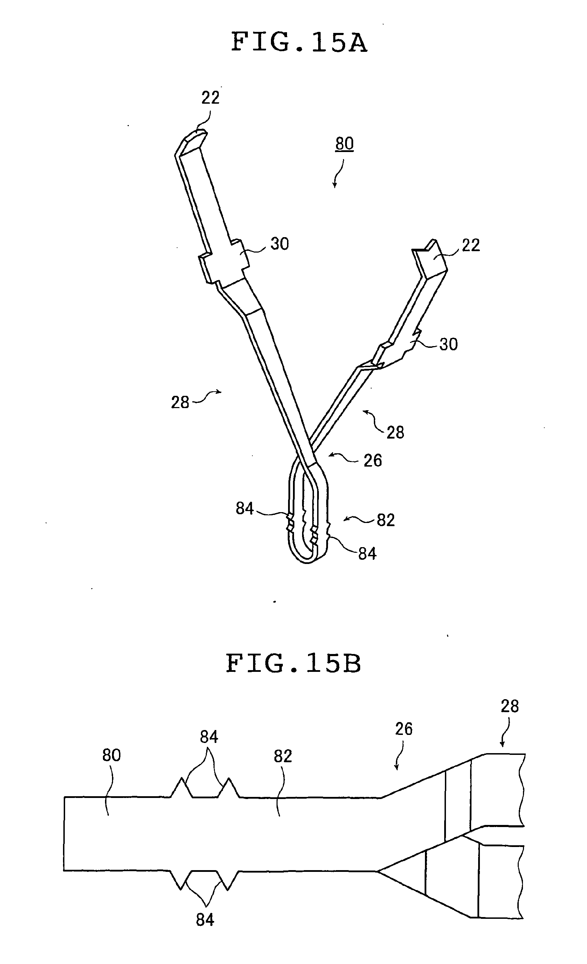

FIG. 15A illustrates a clip 80 used in a magazine type clipping device according to Embodiment 4 of the present invention. The clip 80 is of the same construction as the clip 12 illustrated in FIG. 2 except that a turned portion 82 has four projections 84, and hence the same components are indicated by the same reference symbols, and a detailed description thereof is omitted.

There are provided two projections 84 along each of both end surfaces (side surfaces) of both plate members of the turned portion 82, i.e., eight projections 84 in total. For example, when two consecutive clips 80 are connected together by the connection ring 14, the preceding one of those clips 80 is arranged such that the projections 84 of the turned portion 82 accommodated in the inner hole 43 of the first region 32 of the connection ring 14 are at positions opposed to the skirt portions 38 of the connection ring 14. As illustrated in FIG. 15B, the forward end portions of the projections 84 of the turned portion 82 are formed in a pointed configuration so that they may be engaged in the inner wall surfaces of the skirt portions 38 of the connection ring 14 to thereby reliably fix the skirt portions 38 in position.

Here, when consecutive clips 80 are loaded into the sheath 16 while connected by the connection ring 14, the skirt portions 38 of the connection ring 14 are closed by being pressed by the inner wall of the sheath 16, and enter the inner hole 43 of the first region 32 of the connection ring 14 to abut the projections 84 of the turned portion 82 of the preceding clip 82 accommodated in the first region 32.

Thus, due to its projections 84, the turned portion 82 of the preceding clip 80 is reliably locked by the skirt portions 38 of the connection ring 14, and is reliably retained by the connection ring 14. In this way, the clip 80 is reliably fixed to the connection ring 14. Thus, it is possible to enhance the retention force with which the connection ring 14 retains the clip 80. As a result, even when two clips 80 connected by the connection ring 14 move within the sheath 16 while connected together, the clips 80 can reliably and firmly maintain the connection with the connection ring 14, making it possible to reliably prevent positional deviation between the clips 80 and the connection ring 14.

Further, by turning the two clips 80 connected by the connection ring 14 into an integrated unit, it is possible to achieve an improvement in operability of the magazine type clipping device 10 and perform precision control.

While in the above example two projections 84 are arranged along each edge surface (side end surface) of both plate members of the turned portions 82 of the clip 80, that is, two projections are arranged substantially at the same positions on each edge surface in the pulling direction of the manipulating wire 20, this should not be construed restrictively. There are no particular limitations regarding the formation positions for the projections 84 as long as they are on the edge surfaces of the turned portion 82. Further, the number of projections 84 provided on each edge surface is not restricted to two. It is only necessary for at least one projection to be formed on at least one of both edge surfaces of each plate member of the turned portion 82, and it is also possible to form a different number of projections 84 on each edge surface of each plate member of the turned portion 82. When a large number of projections 84 are formed, the projections are engaged in the inner wall surfaces of the diverged skirt portions 38 to offer resistance to movement when the clip 80 moves in the connection ring 14 at the time of clipping operation, or they generate a large frictional force also when canceling the pressurizing retention by the skirt portions 38. Thus, it is desirable to take this into consideration when setting the number of projections. Further, while in the illustrated example the apexes of the projections 84 are of a pointed configuration, this should not be construed restrictively, and any configuration will do as long as it allows the projections 84 to be engaged in the inner wall surfaces of the skirt portions 38 to exert a predetermined retaining force. Further, while there are no particular limitations regarding the height of the projections 84, it is desirable to adopt a height not causing them to be engaged in the inner wall surfaces of the diverged skirt portions 38 to offer resistance to movement when the clip 80 moves within the connection ring 14 at the time of clipping operation.

While in the above-mentioned embodiments the clips 12 are connected together while alternately changed in orientation by 90 degrees, this should not be construed restrictively. The inner configuration of the connected clips allows selection in conformity with the configuration of the engagement portion. For example, it is also possible to use a clip configured such that twisting is effected by 90 degrees at the portion between the claw portions 22 and the turned portion 24, with the consecutive clips being connected together in the same orientation.

In this case, the positions of the skirt portions 38 of the first region 32 and the positions of the grooves 43a of the second region 34 are deviated from each other, and hence it is desirable for the slits 46 to be provided at positions deviated from the skirt portions 38 in the circumferential direction of the connection ring 14, more preferably, at positions deviated from both the skirt portions 38 and the grooves 43a.

### Embodiment 5

Next, Embodiment 5 of the present invention is described.

While in the embodiments described above the clipping device is placed in the state in which the next clip 12 can be used (standby state) by pulling the sheath 16 to the manipulating portion side, in Embodiment 5, the state in which the next clip 12 can be used is attained by pushing out the manipulating wire 20 to the forward end side.

Here, to be described by way of example is a clipping device in which three clips 12 are loaded for three successive clipping manipulations.

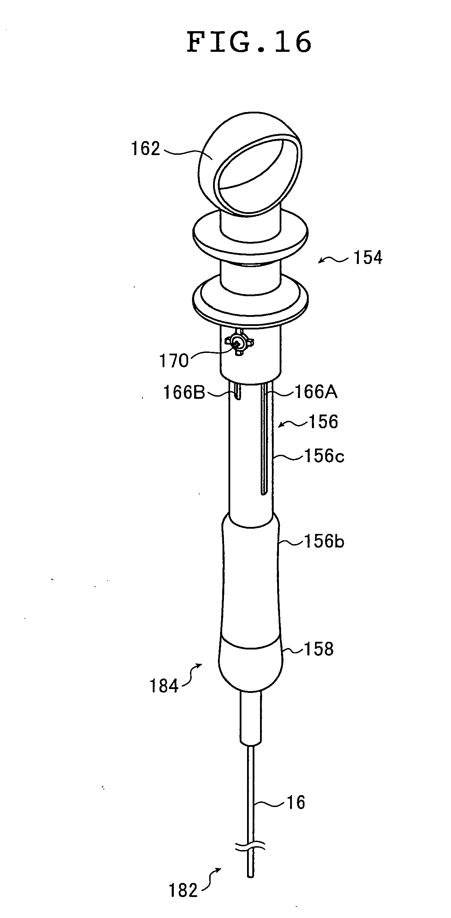

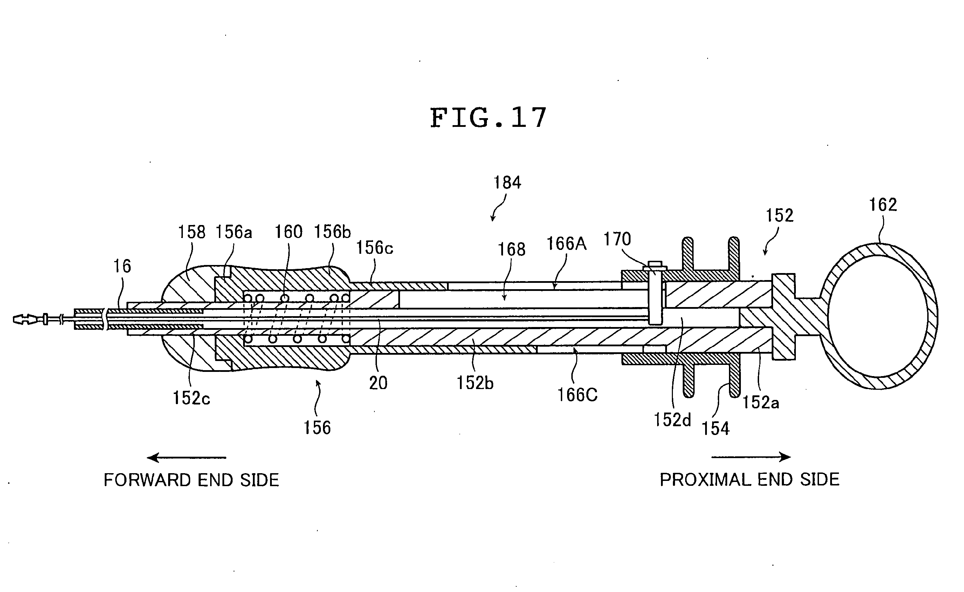

FIGS. 16 and 17 illustrate the construction of a manipulating portion 182 for use in the clipping device of Embodiment 5. The manipulating portion 182 includes the sheath 16, the manipulating wire 20, the connecting member 19 at the forward end of the manipulating wire 20, and a handle portion 184. The handle portion 184 has a handle main body 152, a slider 154, a slider guide 156, a rotating position regulating member 158, an urging spring 160, and a finger hook member 162.

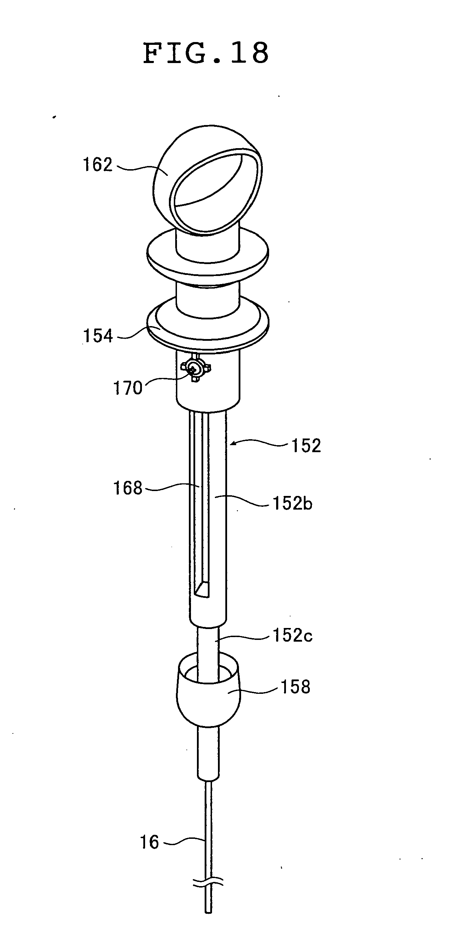

FIG. 18 is a schematic perspective view of the handle main body 152 with the slider guide 156 removed therefrom. The handle main body 152 is a stepped cylindrical member having three cylinder portions differing in outer diameter, and is formed, from the proximal end side, by a large diameter portion 152a, a medium diameter portion 152b, and a small diameter portion 152c.

The handle main body 152 has a through-hole 152d of a fixed diameter extending through the large diameter portion 152a, the medium diameter portion 152b, and the small diameter portion 152c. The finger hook member 162 is fixed to the proximal end side end portion of the large diameter portion 152a by being fixedly fitted into the through-hole 152d. The finger hook member 162 is provided for the doctor to hook his thumb onto it when manipulating the slider 154 described below, and has a ring-like portion.

The medium diameter portion 152b of the handle main body 152 has an engagement groove 168 which is an elongated through-hole extending in the central axis direction of the through-hole 152d. A substantially cylindrical slider guide 156 described below is rotatably inserted into the medium diameter portion 152b.

In the following description, the center axis direction of the cylinder forming the handle main body 152 is referred to as the "axial direction," and the circumferential direction around this axial direction is referred to as the "peripheral direction."

In the handle main body 152, the sheath 16 is fixed to the forward end of the foremost, small diameter portion 152c so as to communicate with the through-hole 152d of the handle main body 152. The manipulating wire 20 is passed through the sheath 16, and protrudes from the proximal end portion of the sheath 16, thereby being passed through the small diameter portion 152c and the medium diameter portion 152b of the handle main body 152 to be connected to the slider 154.

Thus, the sheath 16 is not caused to advance or retreat as in the case of the clipping device 10 illustrated in FIGS. 1A and 1B.

The slider 154 is a substantially cylindrical member which is arranged in the outer periphery of the handle main body 152 so as to pass through the handle main body 152 (and the slider guide 156 described below) and which is movable in the axial direction of the handle main body 152.

The slider 154 has outwardly protruding disc-like flange portions at two positions, that is, the proximal end portion of the cylinder and some midpoint in the axial direction thereof. The operator can hook his finger onto the flange portions and easily move the slider 154 in the axial direction. In an example, the operator inserts his thumb into the ring of the finger hook member 162, and moves the slider 154 in the axial direction while holding the slider 154 between the flange portions between the index finger and the middle finger.

Further, the slider 154 has a slider pin 170 mounted so as to protrude toward the central axis of the handle main body 152. The slider pin 170 passes through the engagement groove 168 to reach the center line of the through-hole 152d of the handle main body 152. Fixed in position in the vicinity of the lower end portion of this slider pin (center line side of the through-hole 152d) is the manipulating wire 20 passed through the smaller diameter portion 152c and the medium diameter portion 152b of the handle main body 152.

As described above, the slider 154 is movable in the axial direction of the handle main body 152. By moving the slider 154, it is possible to cause the manipulating wire 20 inserted into the sheath 16 to advance and retreat (move to the forward end and the proximal end). Through advancement and retreat of the manipulating wire 20 by the slider 154, the clip row at the forward end of the sheath 16 is caused to advance and retreat to place the clipping device in the state in which the next clip 12 can be used.

The position where the proximal end portion of the engagement groove 168 and the slider pin 170 abut each other is the home position (HP) for the slider 154. By moving the slider 154 to the forward end side by a predetermined amount, the manipulating wire 20 is fed toward the forward end side to place the clipping device in the standby state for clipping. By restoring the slider 154 to the HP side from the standby state, the manipulating wire 20 is pulled back, thus effecting clipping and the canceling of the connection between the preceding clip 12 and the succeeding clip 12.

Further, also when loading the clip row into the sheath 16, the slider 154 is moved to the forward end side by a predetermined amount and, in this state, the dummy clip 18 and the manipulating wire 20 are connected together, and the slider 154 is moved to HP, thereby loading the clip row into the sheath 16.

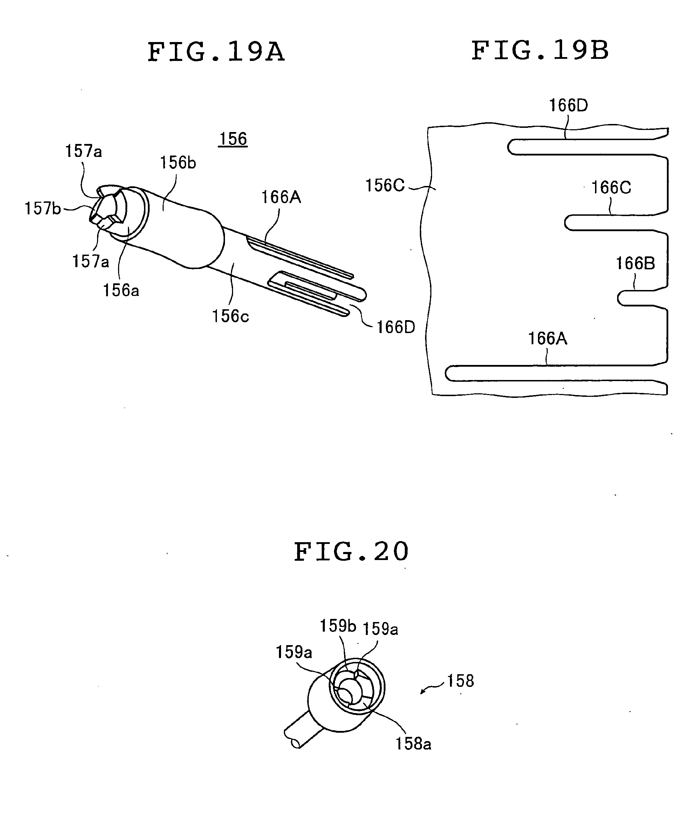

FIG. 19A is a schematic perspective view of a slider guide 156. The slider guide 156 is a substantially cylindrical member for regulating the movement amount in the axial direction of the slider 154, that is, the advancing/retreating amount of the manipulating wire 20 in the longitudinal direction of the sheath 16. The slider guide 156 is supported on the outer peripheral surface of the handle main body 152 so as to be rotatable in the peripheral direction and movable in the axial direction.

The slider guide 156 includes a joint portion 156a, a grasping portion 156b, and a guide portion 156c which are arranged from the forward end side toward the proximal end side and all of which are substantially cylindrical. The slider guide 156 is formed as an integral unit constituting a single cylinder.

The joint portion 156a has an inner diameter substantially equal to the outer diameter of the smaller diameter portion 152c of the handle main body 152, and its convex forward end portion is inserted into a joint portion 158a formed on a rotating position regulating member 158 for regulating the rotating position of the slider guide 156 described below. The joint portion 156a has four protrusions 157a and four recesses 157b between the protrusions 157a, which are formed in a sawtooth-like fashion. The protrusions 157a and the recesses 157b are engaged with protrusions 159a and recesses 159b formed on the joint portion 158a of the rotating position regulating member 158.

The grasping portion 156b is a portion for grasping to allow the operator to rotate the slider guide 156 to effect clipping as described below.

The guide portion 156c has an inner diameter substantially equal to the outer diameter of the medium diameter portion 152b of the handle main body 152, and an outer diameter substantially equal to the inner diameter of the slider 154 and the outer diameter of the large diameter portion 152a of the handle main body 152. Thus, the slider 154 is guided by the large diameter portion 152a of the handle main body 152 and the outer periphery of the guide portion 156c to move in the axial direction.

FIG. 19B is a developed view of the guide portion 156c. The guide portion 156c has axially extending guide grooves 166A through 166D for guiding the slider 154 (slider pin 170). The guide portion 156c has four guide grooves to conform to a clipping device capable of performing clipping three times with the three clips 12 being loaded and without drawing the sheath 16 out of the living body.

In an example, the guide groove 166A corresponds to the loading of the clip row, the guide groove 166B corresponds to the first clipping, the guide groove 166C corresponds to the second clipping, and the guide groove 166D corresponds to the third clipping, with the guide grooves being formed at a circumferential interval of 90 degrees. In the present invention, the number of clips allowing loading (repeating) is not restricted to three, and the guide portion 156c of the slider guide 156 has (n + 1) guide grooves 166, which corresponds to the number n of clips 12 that can be loaded into the clipping device and one guide groove for clip row loading.

The slider grooves 166A through 166D guide the movement of the slider 154 (slider pin 170) together with the engagement groove 168 of the handle main body 12, and, further, regulate the movement amount of the slider 154. By axially reciprocating the slider 154 from HP, there are conducted clipping manipulation and the loading of the clip row (row formed of three clips 12 and the dummy clip 18 connected together by the connection rings 14) into the sheath 16. Further, it is possible to conduct clipping three times without drawing the sheath 16 out of the living body.

The movement amount of the slider 154 differs according to whether the loading of the clip row is conducted and the number of times that clipping has been conducted. In correspondence with this, as illustrated in FIG. 19B, the slider guide 156 has four guide grooves 166A through 166D differing in axial length formed in the guide portion 156c. Thus, the lengths of the guide grooves are lengths through which the slider 154 moves at the time of loading of the clip row and in correspondence with the number of times that clipping is performed.

More specifically, at the time of loading of the clip row, it is necessary for the connecting member 19 to protrude from the sheath 16. Further, in the state in which the slider 154 has been restored to HP, it is necessary for the entire region of the clip row to be accommodated in the sheath 16. Thus, as illustrated in FIG. 19B, the guide groove 166A corresponding to the loading of the clip row is formed in a predetermined length which corresponds to maximum movement amount of the slider 154.

Clipping is performed successively starting with the foremost clip 12. As described below, the HP for the clipping manipulation is the same independently of the number of times that clipping is performed. Thus, the requisite movement amount by which the slider moves from HP toward the forward end in order to place the clipping device in the state in which the next clipping is possible, that is, the state in which the arm portions 28 of the clip 12 and the skirt portions 38 of the connection ring 14 protrude from the forward end of the sheath 16, increases gradually as clipping is performed the first, second and third time.

Thus, as illustrated in FIG. 19B, the guide groove 166B corresponding to the first clipping (clip 12A) is formed in a predetermined length leading to the minimum movement amount of the slider 154. Further, the guide groove 166C corresponding to the second clipping (clip 12B) is formed in a predetermined length leading to the second least movement amount of the slider 154. The guide groove 166C corresponding to the third clipping (clip 12C) is formed in a predetermined length leading to the third least movement amount of the slider 154.

The slider guide 156 is rotated according to the manipulation such as the loading of the clip row and clipping, with each guide groove coinciding with the engagement groove 168 of the handle main body 152. That is, the slider guide 156 is rotated such that the guide groove 166A is matched with the engagement groove 168 at the time of loading of the clip row, that the guide groove 166B is matched with the same at the time of the first clipping (clip 12A), that the guide groove 166C is matched with the same at the time of the second clipping (clip 12B), and that the guide groove 166 is matched with the same at the time of the third clipping (clip 12C).

The four protrusions 157a formed at the forward end of the joint portion 156a are of the same configuration, and the four protrusions 157a are of a sawtooth-like configuration, that is, one tooth surface of each of them is gently tapered, and the other tooth surface thereof exhibits a substantially perpendicular step, thus forming a protrusion of a triangular sectional configuration. The intervals between the adjacent protrusions 157a constitute the recesses 157b. The protrusions 157a and the recesses 157b are engaged with the protrusions 159a and the recesses 159b formed on the joint portion 158a of the rotating position regulating member 158.

The rotating position regulating member 158 is a member arranged on the most proximal side of the handle portion 184, and is a cylindrical member having a cylindrical region and a substantially semi-spherical region, with a through-hole being formed at the center thereof. The rotating position regulating member 158 is fixed to the handle main body 152 by passing the small diameter portion 152c of the handle main body 152 through the through-hole, with the cylindrical region being oriented to the forward end side.

Further, as illustrated in FIG. 20, the rotating position regulating member 158 has a recessed joint portion 158a at the proximal end thereof. As described above, the convex joint portion 156a at the forward end of the slider guide 156 is rotatably inserted into the recessed joint portion 158a.

Like the convex joint portion 156a at the forward end of the slider guide 156, the joint portion 158a has four protrusions 159a of the same configuration which protrude toward the proximal end and which are arranged at equal circumferential intervals, with each of them having two tooth surfaces differing in inclined angle with respect to the abutment surface. The protrusions 159a are formed in a sawtooth-like configuration. That is, one tooth surface of each of them is gently tapered, and the other tooth surface thereof forms a substantially perpendicular, stepped portion, thus forming a protrusion of a triangular sectional configuration. The intervals between the adjacent protrusions 159a are the recesses 159b, which are also four in number.

The protrusions 157a of the joint portion 156a of the slider guide 156 and the recesses 159b of the joint portion 158a of the rotating position regulating member 158 are engaged with each other, and the recesses 157b of the joint portion 156a of the slider guide 156 and the protrusions 159a of the joint portion 158a of the rotating position regulating member 158 are engaged with each other. That is, positioning is effected on the slider guide 156 by the rotating position regulating member 158 at intervals of 90 degrees in the rotating direction.

The guide grooves 166A through 166D of the slider guide 156 are formed such that, when the protrusions and recesses of the joint portion 158a of the rotating position regulating member 158 and the joint portion 156a of the slider guide 156 are engaged with each other, the guide grooves 166A through 166D overlap the engagement grooves 168 of the handle main body 152 in the circumferential direction. That is, the rotation of the slider guide 156 is regulated so as to be stopped by the rotating position regulating member 158 at the position where the guide grooves 166 and the engagement grooves 168 of the handle main body 152 overlap each other.

Each of the protrusions is configured such that one tooth surface has tapered inclined angle and that the other tooth surface is substantially perpendicular, and hence the rotating direction of the slider guide 156 is regulated to one direction. The tooth surfaces of the protrusions are formed such that the guide groove 166A, the guide groove 166B, the guide groove 166C, and the guide groove 166D overlap the engagement groove 168 in that order as the slider guide rotates.

Further, an urging spring 160 is arranged between the step portion between the medium diameter portion 152a and the small diameter portion 152c of the handle main body 152 (i.e., the forward end surface of the medium diameter portion 152b formed by this step portion) and the proximal end surface of the joint portion 156a of the slider guide 156.

The urging spring is a compression spring arranged so as to be wound around the small diameter portion 152c of the handle main body 152. The urging spring exerts an urging force so as to separate the forward end surface of the medium diameter portion 152b and the proximal end surface of the joint portion 156a from each other. That is, the urging spring 160 keeps the slider guide 156 pressed against the rotating position regulating member 158.

Thus, due to the action of the urging spring 160, the slider guide 156 is prevented from being inadvertently rotated.

Further, the slider guide 156 is rotated in a predetermined direction, whereby, due to the protrusions and recesses of the joint portion 158a of the rotating position regulating member 158 and the joint portion 156a of the slider guide 156, the slider guide 156 moves, according to the rotation, toward the proximal end along the tapered portions of the protrusions and recesses of the slider guide 156 against the urging force of the urging spring 160. At the point in time when it is detached from the tapered portions of the protrusions and recesses (the point in time when the protrusions and recesses exhibit substantially perpendicular tooth surfaces), the slider guide 156 moves toward the forward end due to the urging force of the urging spring 160 to be pressed against the rotating position regulating member 158.

As described above, at the position where the protrusions and recesses of the joint portion 158a of the rotating position regulating member 158 and the joint portion 156a of the slider guide 156 are engaged with each other, the engagement groove 168 and the guide grooves 166 are matched with each other in the circumferential direction. Thus, by rotating the slider guide 156, the operator can match the engagement groove 168 with the guide grooves 166 easily and correctly according to the number of times that clipping is performed, etc.

The axial length of the slider guide 156 is set such that, in the state in which it is pressed against the rotating position regulating member 158, there exists, between the step portion between the medium diameter portion 152a and the large diameter portion 152a of the handle main body 152 (i.e., the forward end surface of the large diameter portion 152a formed by this step portion) and the proximal end portion, a gap corresponding to the amount of movement toward the proximal end, etc. due to the protrusions and recesses of the joint portion 158a of the rotating position regulating member 158 and the joint portion 156a of the slider guide 156 at the time of rotation.

With the engagement groove 168 of the handle main body 152 and each guide groove 166 of the slider guide being matched with each other, the slider 154 is moved from HP (position where the proximal end portion of the engagement groove 168 and the slider pin 170 abut each other) to the position where the slider pin abuts the forward end portion of the guide groove 166, and is then returned to HP again, whereby clipping is effected by the clip 12.

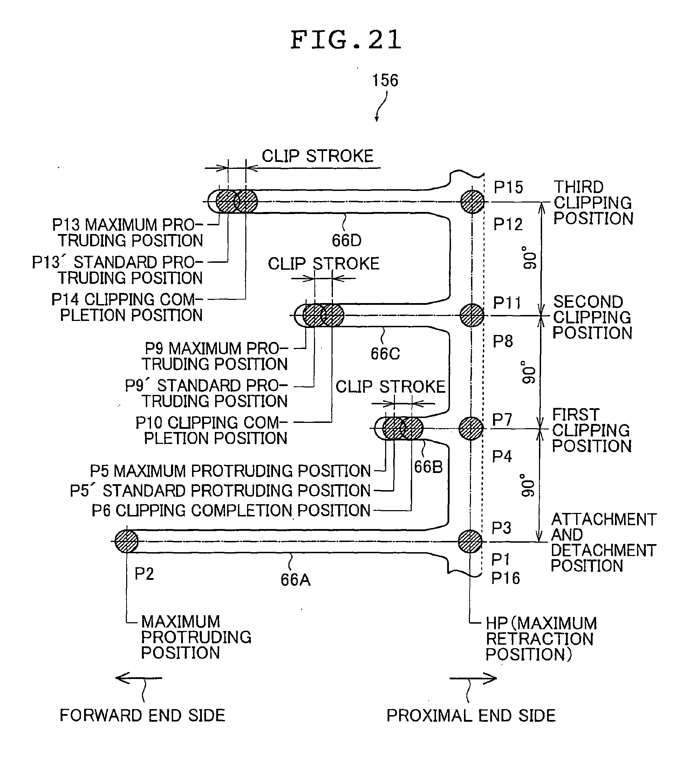

In the following, with reference to FIG. 21, which is a developed view of the slider guide 156, an example of the clipping manipulation conducted three times by the clipping device is described.

First, the slider guide 156 is rotated as needed to match the guide groove 166A with the engagement groove 168 of the handle main body 152, and the slider 154 is moved in the axial direction to HP where the slider pin 170 abuts the forward end surface of the engagement groove 168. That is, the slider pin 170 of the slider 154 is moved to a position P1 illustrated in FIG. 21.

At this time, the forward end of the manipulating wire 20 is retracted into the sheath 16. This state is the initial state of the clipping by the clipping device.

In the present invention, instead of causing the forward end surface of the engagement groove 168 and the slider pin 170 to abut each other, it is also possible to cause the main body of the slider 154 and the forward end surface of the engagement groove 168 to each other, thereby regulating the movement amount in the axial direction of the slider 154.

Next, the slider 154 is moved to the position where it abuts the forward end portion of the guide groove 166A, that is, the slider pin 170 is moved to a maximum protruding position P2. As a result, the forward end of the manipulating wire 20 protrudes by a predetermined amount from the forward end of the sheath 16.

In this state, the connecting member 19 of the dummy clip 18 is attached to the forward end of the manipulating wire 20. As a result, a clip row formed of the three clips 12 and the dummy clip 18 connected together by the connection rings 14 is connected to the manipulating wire 20.

Next, the slider pin 170 is restored to a position P3 illustrated in FIG. 21, that is, to HP. Through this manipulation, the clip row is accommodated in the sheath 16. As a result, the loading of the clip row formed of the clips 12 connected together into the manipulating portion 182 is completed.

After that, the sheath 16 is inserted into the port of the forceps of the endoscope or the like inserted into the living body. Then, the forward end of the sheath 16 is caused to reach the forward end of the insert portion of the endoscope, and is then caused to protrude from the forward end of the endoscope. Further, through manipulation of the insert portion or the angle portion of the endoscope, the forward end of the sheath 16 is moved to the target position.

When the requisite manipulation has been completed, the slider guide 156 is rotated by 90 degrees to match the guide groove 166B with the engagement groove 168. As a result, the position of the slider pin 170 is moved to a position P4 in FIG. 21, that is, HP, which corresponds to the guide groove 166B.

Next, the slider 154 is moved to the position where it abuts the forward end portion of the guide groove 166B, that is, to a maximum protruding position P5 in FIG. 21. Through this extrusion of the slider 154, that is, the extrusion of the manipulating wire 20, the clip row is moved in the direction of the forward end, and the foremost clip 12A and the first region 32 of the connection ring 14A protrude from the forward end of the sheath 16. As a result, the arm portions 28 of the clip 12A are opened, and further, the skirt portions 38 of the connection ring 14A are opened.

It should be noted that there is dimensional variation or the like due to a production error in the clips 12 and the connection rings 14. Further, in the clipping device inserted into the endoscope, there may be a case in which the protruding amount of the manipulating wire 20 decreases due to a difference between the inner and outer periphery, etc. attributable to bending, curving, etc. of the manipulating wire 20 and the sheath 16. Thus, the forward end of the guide groove 166B is at the maximum protruding position P5 where the clip 12A is not detached from the sheath 16, and where the skirt portions 38 of the connection ring 14A are reliably opened independently of a production error in the clips 12, etc. or the condition of the sheath 16.

Thus, normally, in the state in which the slider pin 170 has been pushed forward to the maximum protruding position P5, the skirt portions 38 of the connection ring 14A are situated in front of the forward end portion of the sheath 16, and the skirt portions 38 and the sheath 16 are spaced apart from each other.

This also applies to the forward end portion of the guide groove 166C corresponding to the second clipping by the clip 12B, and to the forward end portion of the guide groove 166D corresponding to the third clipping by the clip 12C.

Next, while watching, for example, the display of the endoscope, the operator restores the slider pin 170 to the HP side, and restores the clip row to the sheath 16 to the standard protruding position P5' where the skirt portions 38 of the connection ring 14A abut the forward end portion of the sheath 16. As a result, the preparation for the first clipping (clipping by the first clip 12) is completed.

After that, the endoscope is operated to press the claw portions 22 of the diverged clip 12A against the portion of the living body to be subjected to clipping, and, in this state, the slider pin 170 is moved to the proximal side to be restored to HP, that is, the position P7.

Through this movement of the slider pin 170, the foremost clip 12A is drawn into the connection ring 14A, and the arm portions 28, which have been open, are closed by the clamping ring 40, with the claw portions 22 being closed to effect clipping on the living body. When the slider pin 170 moves from the standard protruding position P5' to the clipping completion position P6, the portions of the arm portions 28 directly below the projections 30 are drawn into the connection ring 14A, whereby the clipping is completed.

Simultaneously with the completion of the clipping, the proximal end portion of the foremost clip 12A (proximal end portion of turned portion 24) and the claw portions 22 of the second clip 12B are discharged from the proximal end portion of the connection ring 14A. As a result, the arm portions 28 of the second clip 12B, which have been closed by the second region 34 of the connection ring 14A, are opened up to the inner diameter of the sheath 16, and the engagement between the turned portion 24 of the preceding clip 12A and the claw portions of the next clip 12B is released, whereby the clip 12A and the connection ring 14A are separated from the clip row, thereby attaining the state in which the clip 12A and the connection ring 14A can be discharge from the sheath 16.

Further, in the state in which the slider pin 170 has been restored to the position P7, the clip row separated from the clip12A and the connection ring 14A is drawn into the sheath 16.

As is apparent from the above description, the distance between the maximum protruding position P5 (P9, P13) and the standard protruding position P5' (P9', P13') serves as a buffer for absorbing a production error in the components, a difference between the inner and outer periphery of the sheath 16, etc. Thus, by once pushing out the slider pin 170 to the maximumprotruding position P5, it is possible to reliably open the arm portions 28 and the skirt portions 38 to perform clipping independently of the production error in the clips 12 or the condition of the sheath 16 in the living body.

In a preferable manipulation, the slider pin 170 is pushed out to the maximum protruding position P5, and then returned to the standard protruding position P5'. After that, the claw portions 22 are brought into contact with the living body to effect clipping (restoration of the slider 54 to P7, which is HP), whereby it is possible to more reliably prevent detachment, etc. of the clip 12 attributable to excessive protrusion from the sheath 16. Further, it is possible to press the reliably retained clip 12 firmly against the living body to be subjected to clipping.

When, at the maximum protruding position, the foremost clip 12 is firmly retained, and there is no (or very little) risk of detachment, the slider pin 170 may be pulled back at a stroke from the maximum protruding position to HP to effect clipping and the releasing of the connection of the clip row.

It is also desirable to generate a small impact (i.e., so-called click feel) by well-known means such as a protrusion and a recess engaged with each other or an urged spherical body and a recess engaged therewith at the point in time when the slider pin 170 passes the clipping completion position P6 (P10, P14), thus enabling the operator performing the clipping to be aware of the completion of the clipping.

When the slider pin 170 has been restored to the position P7, which is HP, to complete the first clipping (clipping by the first clip 12A), the slider guide 156 is rotated by 90 degrees as illustrated in FIG. 12 (H) to match the guide groove 166C with the engagement groove 168. As a result, the position of the slider pin 170 moves to HP, which corresponds to the guide groove 166C, as indicated at P8 in FIG. 21.

Next, the slider pin 170 is moved to the maximum protruding position P9 where the slider pin 170 abuts the forward end portion of the guide groove 166C. Through this manipulation, the second clip 12B and the first region 32 of the connection ring 14B protrude from the forward end of the sheath 16, with the arm portions 28 and the skirt portions 38 opening. Further, by pulling the slider pin 170 back to the standard protruding position P9' where the skirt portions 38 abut the forward end of the sheath 16, the clipping device is placed in the state in which the clipping device is ready for the second clipping (by the clip 12B).

When the clipping device has become ready for clipping, the claw portions 22 of the diverged clip 12B are pressed against the portion which is to be subjected to clipping, and the slider pin 170 is moved to the proximal side to be pulled back to HP, that is, the position P11.

As a result, through the movement of the slider pin 170 from the standard protruding position P9' to the clipping completion position P10, the clipping by the second clip 12 is completed, and the second clip 12B and the next clip 12C (one on the most proximal side) are separated from each other, whereby a state is attained in which the clip 12B and the connection ring 14B can be discharged from the sheath 16.

In the state in which the slider pin 170 has been restored to the position P11, which is HP, the clip row separated from the clip 12B and the connection ring 14B is in the state in which the clip row has been drawn into the sheath 16.

When the second clipping is completed, the slider guide 156 is then rotated by 90 degrees to match the guide groove 166D with the engagement groove 168. As a result, the position of the slider pin 170 moves to HP, which corresponds to the guide groove 166D, indicated as the position P12 in FIG. 21.

Next, the slider pin 170 is moved to the maximum protruding position P13 where the slider pin 170 abuts the forward end portion of the guide groove 166D. Through this manipulation, the third clip 12C and the connection ring 14B protrude from the forward end of the sheath 16, with the arm portions 28 and the skirt portions 38 opening. Further, by pulling the slider pin 170 back to the standard protruding position P13', the clipping device is placed in the state in which the clipping device is ready for the third clipping.

When the clipping device has become ready for clipping, the claw portions 22 of the diverged clip 12C are pressed against the portion which is to be subjected to clipping, and the slider pin 170 is moved to the proximal side to be pulled back to HP, that is, the position P15.

As a result, clipping is performed in the same manner as described above, and the clipping by the third clip 12C is completed through the movement of the slider pin 170 from the standard protruding position P13' to the clipping completion position P14, and, further, the third clip 12C and the dummy clip 18 are separated from each other, whereby the state is attained in which the clip 12C and the connection ring 14C can be discharged from the sheath 16.

In the state in which the slider pin 170 has been restored to the position P15, which is HP, the dummy clip 18 separated from all the clips is in the state in which the dummy clip 18 has been drawn into the sheath 16.

When the clipping by the three clips 12 has been completed, the slider guide 156 is rotated by 90 degrees to match the guide groove 166A with the engagement groove 168. As a result, the position of the slider pin 170 is restored again to HP, which corresponds to the guide groove 166A as indicated by the position P1 in FIG. 21. After that, the sheath 16 is pulled out of the endoscope.

After the sheath 16 has been pulled out, the slider pin 170 is pushed out to the position P2 where the slider pin 170 abuts the forward end portion of the guide groove 166A, and the dummy clip 18 and the connecting member 19 are caused to protrude from the forward end of the sheath 16, thereby removing the dummy clip 18 and the connecting member 19 from the forward end of the manipulating wire 20.

As described above, it is possible to perform clipping a plurality of times without pulling out the sheath. Further, solely through the rotation of the slider guide 156 and the reciprocating movement of the slider 154, the clip row is moved in the axial direction (longitudinal direction of the sheath 16) by a proper amount according to the number of times that clipping is performed (first time, second time...) to place the clipping device in the state in which the clipping device is ready for clipping, making it possible to perform clipping and the separation of the clips connected together. That is, it is possible to perform accurate clipping through easy manipulation.

The clipping device and the method of loading the connected clips of the embodiments of the present invention described in detail above should not be construed restrictively. It goes without saying that various improvements and variations are possible without departing from the gist of the present invention. The clipping device of the present invention is applicable not only to a soft endoscope but also to a hard endoscope.

## Claims

1. A magazine type clipping device comprising:
a plurality of clips loaded into a forward end portion of a sheath while being engaged with other clips longitudinally connected together;
at least one connection ring fitted into the sheath so as to be capable of advancing and retreating, and covering an engagement portion of the clips to maintain the clips in a connected state; and
a manipulating wire connected to a rearmost clip and adapted to pull a clip row formed of the plurality of clips,
wherein each of the plurality of clips has a pair of openable/closable claw portions and a tail portion,
wherein the connection ring has a connection maintaining region retaining a pair of the plurality of clips longitudinally engaged with each other, with the pair of claw portions of a succeeding clip being closed while holding the tail portion of a preceding clip, and
wherein the engagement portion between the pair of the plurality of clips is detached from the connection maintaining region through an operation of pulling the manipulating wire to open the pair of claw portions of the succeeding clip, with the tail portion of the preceding clip being detached from therebetween.

2. The magazine type clipping device according to Claim 1, wherein the connection ring has, at the same position in a clip pulling direction and at positions circumferentially spaced apart from each other, two or more skirt portions which, when being situated inside the sheath, are pressed by an inner wall of the sheath to be inwardly closed to thereby pressurize at least one of the pair of the plurality of clips engaged with each other within the connection ring, and which, when being situated outside of a forward end of the sheath, are opened in a width larger than an inner diameter of the sheath to prevent retreat into the sheath.

3. The magazine type clipping device according to Claim 1 or 2, wherein the connection ring has a clamping portion arranged on a forward end side thereof and, when being situated on a forward end side of the succeeding clip, abutting the succeeding clip to clamp the succeeding clip so as to close the pair of claw portions of the succeeding clip.

4. The magazine type clipping device according to any one of Claims 1 through 3, wherein the plurality of clips are connected with each other, with orientations thereof being alternately changed by 90 degrees.

5. The magazine type clipping device according to any one of Claims 1 through 4, wherein, after the foremost clip has been used for clipping, the sheath is moved relative to the manipulating wire to a position where the succeeding clip protrudes from the sheath so that the succeeding clip becomes usable.

6. The magazine type clipping device according to any one of Claims 1 through 5, wherein the connection ring is formed of a resin material.

7. The magazine type clipping device according to any one of Claims 1 through 6, wherein the connection ring has a connection canceling region arranged on a proximal end side of the connection maintaining region and adapted to provide an interval between the pair of the claw portions of the succeeding clip when the pair of the claw portions of the succeeding clip are opened to allow detachment of the tail portion of the preceding clip:

8. The magazine type clipping device according to Claim 7, wherein the foremost clip is pulled by the manipulating wire relative to the connection ring corresponding thereto, with the engagement portion between the foremost clip and the succeeding clip being detached from the connection maintaining region of the connecting ring, and hence connection of the foremost clip with the succeeding clip can be canceled, and the succeeding clip is then detached from the connection canceling region of the connection ring so that the foremost clip is detached from the succeeding clip.

9. A magazine type clipping device comprising:
a plurality of clips loaded into a forward end portion of a sheath while being engaged with other clips longitudinally connected together;
at least one connection ring fitted into the sheath so as to be capable of advancing and retreating, and covering an engagement portion of the clips to maintain the clips in a connected state; and
a manipulating wire connected to a rearmost clip and adapted to pull a clip row formed of the plurality of clips,
wherein the connection ring has at least one slit extending from a proximal end thereof to a position on a proximal end side of the engagement portion between the plurality of clips.

10. The magazine type clipping device according to Claim 9, wherein the connection ring has two or more slits.

11. The magazine type clipping device according to Claim 9 or 10,
wherein the connection ring has two or more skirt portions, which, when being situated inside the sheath, are pressed by an inner wall of the sheath to be inwardly closed to thereby pressurize at least one of a pair of the plurality of clips engaged with each other within the connection ring.

12. The magazine type clipping device according to Claim 11, wherein the skirt portions of the connection ring are arranged at the same position in a clip pulling direction and at positions circumferentially spaced apart from each other, and, when being situated outside of a forward end of the sheath, are opened in a width larger than an inner diameter of the sheath to prevent retreat into the sheath.

13. The magazine type clipping device according to Claim 11 or 12, wherein the slits are formed at positions different from positions of the skirt portions in a circumferential direction of the connection ring.

14. The magazine type clipping device according to any one of Claims 11 through 13, wherein each of the plurality of clips comprises:
a pair of claw portions opposed to each other at one end and capable of being opened and closed;
two arm portions supporting the pair of claw portions and crossing each other;
a turned portion connecting the two arm portions at another end; and
at least one projection formed around the turned portion and engaged with the skirt portions of the connection ring.

15. The magazine type clipping device according to any one of Claims 9 through 14, wherein the foremost clip is pulled with respect to the connection ring corresponding by the manipulating wire, and an engagement portion between the foremost clip and a succeeding clip is detached from the connection ring to thereby cancel connection of the foremost clip with the succeeding clip.

16. The magazine type clipping device according to any one of Claims 9 through 15, wherein, after the foremost clip has been used for clipping, the sheath is moved relative to the manipulating wire to a position where the succeeding clip protrudes from the sheath so that the succeeding clip becomes usable.

17. The magazine type clipping device according to any one of Claims 9 through 16, wherein the connection ring comprises:
a resin retaining portion maintaining the plurality of clips in the connected state; and
a metal clamping portion arranged on a forward end side of the retaining portion and, when being situated on a forward end side of the succeeding clip, abutting the succeeding clip to clamp the succeeding clamp so as to close the pair of claw portions.

18. The magazine type clipping device according to any one of Claims 9 through 17, wherein the plurality of clips are connected with each other, with orientations thereof being alternately changed by 90 degrees.
